(19) **Europäisches Patentamt / European Patent Office / Office européen des brevets**

(11) **EP 2 659 870 B1**

(12) **EUROPEAN PATENT SPECIFICATION**

(45) Date of publication and mention
of the grant of the patent:
**02.05.2018 Bulletin 2018/18**

(21) Application number: **11852337.2**

(22) Date of filing: **26.12.2011**

(51) Int Cl.:
*A61F 13/496* (2006.01)      *A61F 13/15* (2006.01)
*A61F 13/49* (2006.01)

(86) International application number:
**PCT/JP2011/080060**

(87) International publication number:
**WO 2012/090931 (05.07.2012 Gazette 2012/27)**

(54) **UNDERWEAR-TYPE ABSORBENT ARTICLE**

SAUGFÄHIGER ARTIKEL VOM UNTERWÄSCHE-TYP

ARTICLE ABSORBANT DE TYPE SOUS-VÊTEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2010   JP 2010291047
27.12.2010   JP 2010291048**

(43) Date of publication of application:
**06.11.2013   Bulletin 2013/45**

(73) Proprietor: **Kao Corporation
Chuo-Ku
Tokyo 103-8210 (JP)**

(72) Inventors:
• **ONDA, Aiko
Haga-gun
Tochigi 321-3497 (JP)**

• **SASAKI, Jun
Haga-gun
Tochigi 321-3497 (JP)**

(74) Representative: **Vossius & Partner
Patentanwälte Rechtsanwälte mbB
Siebertstrasse 3
81675 München (DE)**

(56) References cited:
**WO-A1-2008/108270      WO-A1-2009/119195
WO-A1-2011/001937      WO-A1-2011/001944
WO-A1-2011/043373      CN-A- 101 773 432
JP-A- 8 038 546           JP-A- 2006 181 172
JP-A- 2007 509 725      JP-A- 2008 178 682
JP-A- 2009 160 128      JP-A- 2009 160 129
JP-A- 2009 240 640      JP-A- 2009 240 694
JP-A- 2009 240 695**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a pull-on absorbent article such as a disposable diaper.

Background Art

Conventionally, there has been known a pull-on absorbent article including an external material shaped like a sandglass extended over a front portion to be worn about the front of a wearer, a crotch portion to be worn about the crotch of a wearer, and a rear portion to be worn about the back of a wearer, and an absorbent assembly which is fixed to the inner face side of the external material. Here, both side edge portions of the external material at the front portion and both side edge portions of the external material at the rear portion are joined, so that a waist opening and a pair of leg openings are formed.

When continuously manufacturing such underwear-type articles, it is common that penetrating holes or cutouts for forming leg openings are formed at a continuous-length original material of the external material and that unnecessary portions are trimmed and eliminated.

Further, as a conventional pull-on absorbent article, there has been known a pull-on absorbent article in which an external material is divided into a front panel to be worn about the front of a wearer and a rear panel to be worn about the back of a wearer, an absorbent assembly is fixed as bridging between the front panel and the rear panel, and both lateral side edge portions of the front panel and both lateral side edge potions of the rear panel are joined.

For example, Patent Literature 1 discloses a pull-on clothing article as a pull-on absorbent article which includes an absorbent assembly and a circular elastic belt structured with a front belt portion and a rear belt portion. Here, a length of the rear belt portion (rear panel) in the longitudinal direction is longer than a length of the front belt portion (front panel) in the longitudinal direction.

Further, Paten Literature 2 discloses an underwear-type paper diaper including an absorbent assembly and a cylinder-shaped waist portion which is formed with a front external sheet and a rear external sheet. Here, the rear external sheet includes a rear body portion and a rear extension portion extended downward therefrom. Then, a stretch rate of a second elastic stretchable member arranged at a buttock cover portion extended to both sides of a portion of the rear extension portion overlapped with the absorbent assembly is set to be higher than a stretch rate of a first elastic stretchable member arranged at an intermediate part and a lower end part of the rear body portion.

Further, Patent Literature 3 also discloses an underwear-type paper diaper including an absorbent assembly and a cylinder-shaped waist portion which is formed with a front external sheet and a rear external sheet. Here, the rear external sheet includes a rear body portion and a rear extension portion extended downward therefrom. According to the disclosure, it is also possible that the front external sheet includes a front body portion and a front extension portion.

Citation List

Patent Literature

Patent Literature 1: JP 2008-508082 A
Patent Literature 2: JP 2008-178682 A
Patent Literature 3: JP 2008-212249 A

Summary of Invention

For example, a continuous-length original material for a front panel and a continuous-length original material for a rear panel are conveyed as being spaced and absorbent bodies are intermittently fixed as bridging between both thereof. Subsequently, joining for forming side seals is performed after two-folding causing the continuous-length original material for the front panel and the continuous-length original material for the rear panel to be overlapped. At the same time with or after the joining, cutting is performed to divide the above into an individual disposable diaper. In this manner, a pull-on absorbent article in which an external material is divided into the front panel adopted to be worn the wearer's front side and the rear panel adopted to be worn about the wearer's back side can be manufactured effectively. Compared to a case to form penetrating holes or cutouts for forming leg openings at the wide external material in which a portion adopted to be worn about the front side and a portion adopted to be worn about the back side are continued, according to this method, trimming of an original material of the external material can be eliminated or portions to be trimmed can be lessened.

The pull-on clothing article disclosed in Patent Literature 1 excellently covers the buttocks owing to that the rear belt portion (rear panel) is longer than the front belt portion (front panel). However, the extension portion (rear extension portion) to cover the buttocks has inferior fit to skin and inconvenience such that the portion is turned up is prone to be caused. Further, since an extension portion does not exist at the front side, a problem in appearance is caused by insufficient covering of skin as a clothing article while worn.

In the underwear-type paper diapers of Patent Literatures 2 and 3, since a lower end of the rear panel is processed to be curved, trimming is required at the time of manufacturing and covering the buttocks is inferior.

Currently, there is no suggestion against a problem that slip-drop is prone to occur while worn with a conventional pull-on absorbent article in which an external material is divided into a front panel and a rear panel.

Accordingly, the present invention relates to a pull-on absorbent article which excellently covers the buttocks and causes less slip-drop and appearance worsening while worn even being a pull-on absorbent article capable of being effectively manufactured with an external material divided into a front panel and a rear panel.

Further, as described above, a conventional pull-on absorbent article in which an external material is divided into a front panel and a rear panel has a problem that slip-drop is prone to occur while worn. Further, since an extension portion does not exist at the front side, a problem in appearance is caused by insufficient covering of skin as a clothing article while worn.

According to findings of the present inventors with studies to solve the abovementioned problems, slip-drop of a diaper can be effectively prevented by increasing contraction stresses in the article lateral direction of the rear panel and the front panel, in particular, at regions in the vicinity of a lower end part (an end part at the crotch portion side) of the side seal. However, in a case that large contraction stresses to be capable of preventing slip-drop are provided only to the regions, there arises a problem of a burden to skin such that the regions are strongly contacted to skin and rubber imprints are left thereat.

Accordingly, the present invention relates to a pull-on absorbent article having a small burden to skin with pressing imprints of elastic members less likely to be left as excellently preventing slip-drop even being a pull-on absorbent article with an external material divided into a front panel and a rear panel.

A pull-on absorbent article of the present invention having a waist opening and a pair of leg openings includes a rectangular front panel adapted to be worn about the front of a wearer, a rectangular rear panel adapted to be worn about the back of a wearer, and an absorbent assembly fixed as bridging between the front panel and the rear panel. Each of the front panel and the rear panel includes a body portion (a front body portion, a rear body portion) which includes side seals at both lateral sides and an extension portion (a front extension portion, a rear extension portion) which is extended from the body portion toward a crotch portion without including the side seals at both lateral sides. Hereinafter, the above structure is called a basic structure as well.

In addition to the basic structure, a pull-on absorbent article of the present invention has a structure (A) and/or a structure (B) described below.

(A) Each of the body portion (front body portion) and the extension portion (front extension portion) of the front panel and the body portion (rear body portion), an upper rear extension portion, and a lower rear extension portion of the rear panel has stretchability in the article lateral direction while the extension portion (rear extension portion) of the rear panel is sectionalized into the upper rear extension portion and the lower rear extension portion by bisecting the extension portion (rear extension portion) of the rear panel in the article longitudinal direction. Further, each of the upper rear extension portion and a portion of the front panel corresponding to the upper rear extension portion has a larger contraction stress in the article lateral direction per unit length in the article longitudinal direction than a contraction stress of the lower rear extension portion.

(B) The extension portion (rear extension portion) of the rear panel has a length in the article longitudinal direction being the same as or larger than that of the extension portion (front extension portion) of the front panel. Each of the body portion (front body portion) of the front panel, the front extension portion, the body portion (rear body portion) of the rear panel, and a rear corresponding portion of the rear extension portion corresponding to the front extension portion has stretchability in the article lateral direction owing to each elastic member arranged in the article lateral direction.

Further, following relational expressions (11) to (14) are satisfied under definitions that $P_{A1} \sim P_{A4}$ denote contraction stresses in the article lateral direction per unit length in the article longitudinal direction and $Q_{A1} \sim Q_{A4}$ denote contraction stresses in the article lateral direction per single elastic member respectively of first to third regions A1' to A3' and the front extension portion 21a (A4') of the front panel, while the front body portion is sectionalized into three regions by trisecting the side seal in the article longitudinal direction to be the first region A1', the second region A2', and the third region A3' sequentially from a side close to the waist opening and definitions that $P_{B1} \sim P_{B4}$ denote contraction stresses in the article lateral direction per unit length in the article longitudinal direction and $Q_{B1'} \sim Q_{B4'}$ denote contraction stresses in the article lateral direction per single elastic member respectively of first to third regions B1' to B3' and the rear corresponding portion B4' of the rear panel, while the rear body portion is sectionalized into three regions by trisecting the side seal in the article longitudinal direction to be the first region B1', the second region B2', and the third region B3' sequentially from a side close to the waist opening.

$$\text{Ratio } (P_{A3'}/P_{A1'}) > \text{Ratio } (Q_{A3'}/Q_{A1'}), \text{ Ratio } (P_{A3'}/P_{A1'}) > 1 \ldots (11)$$

$$\text{Ratio } (P_{B3'}/P_{B1'}) > \text{Ratio } (Q_{B3'}/Q_{B1'}), \text{ Ratio } (P_{B3'}/P_{B1'}) > 1 \ldots (12)$$

$$\text{Ratio } (P_{A4'}/P_{A1'}) > \text{Ratio } (Q_{A4'}/Q_{A1'}), \text{ Ratio } (P_{A4'}/P_{A1'}) > 1 \ldots (13)$$

$$\text{Ratio } (P_{B4'}/P_{B1'}) > \text{Ratio } (Q_{B4'}/Q_{B1'}), \text{ Ratio } (P_{B4'}/P_{B1'}) > 1 \ldots (14)$$

Brief Description of Drawings

[FIG. 1] FIG. 1 is a perspective view illustrating a usage state (worn state) of a disposable pull-on diaper being a first embodiment of the present invention.
[FIG. 2] FIG. 2 is a partially-sectioned plane view illustrating an opened and extended state of the disposable pull-on diaper illustrated in FIG. 1. Here, the opened and extended state denotes a state that joint portions (side seals) at both sides of a diaper are torn off to put a pull-on absorbent article into an opened state and that the absorbent article in the opened state is extended to have designed dimensions (same as dimensions when being planarly extended with influences of elastic members completely eliminated) with the respective elastic members extended.
[FIG. 3] FIG. 3 is a partially-sectioned enlarged view of a front portion side of the disposable pull-on diaper illustrated in FIG. 1 in a stretched state viewing from a diaper outer face side.
[FIG. 4] FIG. 4 is a partially-sectioned enlarged view of a rear portion side of the disposable pull-on diaper illustrated in FIG. 1 in a stretched state viewing from a diaper outer face side.
[FIG. 5] FIG. 5 shows sectional views of each stretchable portion along the diaper lateral direction in the disposable pull-on diaper illustrated in FIGs. 1 and 6. FIG. 5(a) is a view illustrating a state that gathers are eliminated by extending the elastic members. FIG. 5(b) is a view illustrating a state that gathers are formed with contraction of the elastic members.
[FIG. 6] FIG. 6 is a perspective view illustrating a usage state (worn state) of a disposable pull-on diaper being a second embodiment of the present invention.
[FIG. 7] FIG. 7 is a partially-sectioned plane view illustrating an opened and extended state of the disposable pull-on diaper illustrated in FIG. 6.
[FIG. 8] FIG. 8 is a partially-sectioned enlarged view of a front portion side of the disposable pull-on diaper illustrated in FIG. 6 in a stretched state viewing from a diaper outer face side.
[FIG. 9] FIG. 9 is a partially-sectioned enlarged view of a rear portion side of the disposable pull-on diaper illustrated in FIG. 6 in a stretched state viewing from a diaper outer face side.

Description of Embodiments

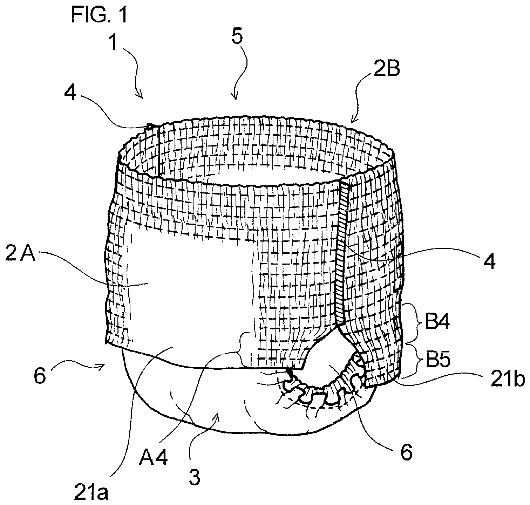

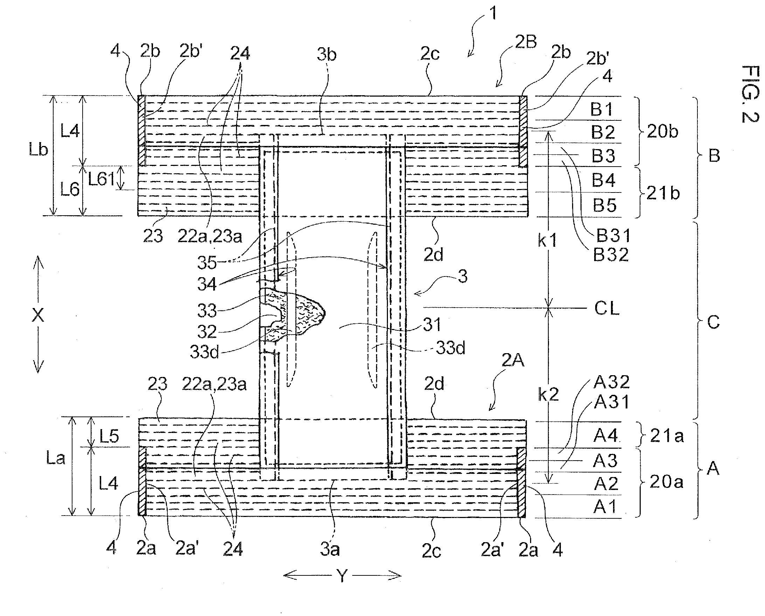

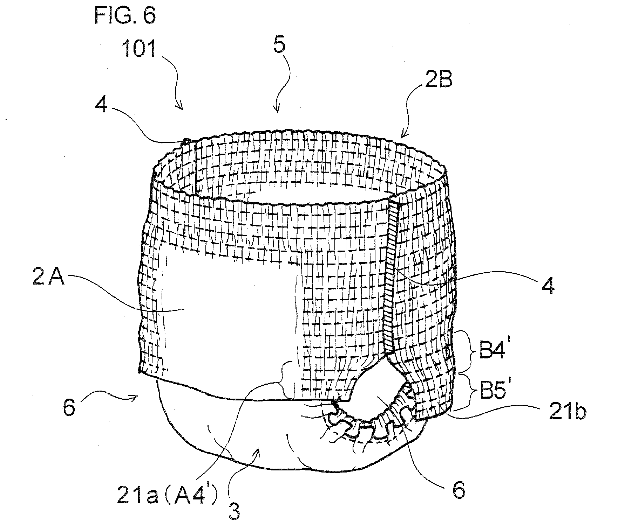

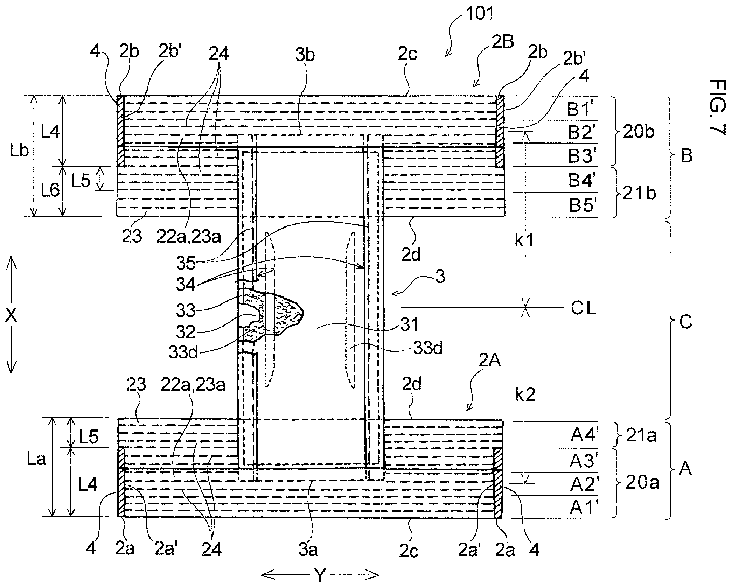

In the following, a pull-on absorbent article of the present invention will be described based on preferable embodiments with reference to the drawings.
As illustrated in FIGs. 1 and 2, a disposable pull-on diaper 1 (hereinafter, simply called the diaper 1) of a first embodiment of the present invention includes a rectangular front panel 2A adapted to be worn around a wearer's front, a rectangular rear panel 2B adapted to be worn around a wearer's back, and an absorbent assembly 3 fixed to the front panel 2A and the rear panel 2B so as to bridge them. A pair of side seals 4, 4 is formed by joining both side edge portions 2a, 2a of the front panel 2A and both side edge portions 2b, 2b of the rear panel 2B.
As illustrated in FIGs. 6 and 7, a disposable pull-on diaper 101 (hereinafter, simply called the diaper 101) of a second embodiment of the present invention includes a rectangular front panel 2A adapted to be worn around a wearer's front, a rectangular rear panel 2B adapted to be worn around a wearer's back, and an absorbent assembly 3 fixed to the front panel 2A and the rear panel 2B so as to bridge them. A pair of side seals 4, 4 is formed by joining both side edge portions 2a, 2a of the front panel 2A and both side edge portions 2b, 2b of the rear panel 2B.

Structures common to the diapers 1, 101 of the first and second embodiments will be described.

As illustrated in FIGs. 1 and 2 or FIGs. 6 and 7, each of the diapers 1, 101 of the first and second embodiments includes a front portion A adapted to be worn about the front of a wearer, a rear portion B adapted to be worn about the back of a wearer, and a crotch portion C located between the front portion A and the rear portion B and adapted to be worn about the crotch of a wearer. A diaper longitudinal direction (article longitudinal direction) denotes a direction from the front portion A to the rear portion B via the crotch portion C or a direction opposite thereto (direction X in FIGs. 2 and 7). A diaper lateral direction (article lateral direction) denotes a direction along the waist line of a wearer (direction Y in FIGs. 2 and 7) and a direction perpendicular to the article longitudinal direction. In the following, the diaper longitudinal direction (article longitudinal direction) and the diaper lateral direction (article lateral direction) may be simply called direction X and direction Y respectively, as well.

As illustrated in FIG. 2 or FIG. 7, in the side seal 4 of the diaper 1, 101, a length in direction X is smaller than both of a length La of the front panel 2A and a length Lb of the rear panel 2B. A body portion 20a, 20b having the side seals 4, 4 at both lateral sides and an extension portion 21a, 21b which is extended from the body portion 20a, 20b toward the crotch portion C and which has no side seals 4, 4 at both lateral sides are formed at each of the front panel 2A and the rear panel 2B.

In the following, the body portion 20a and the extension portion 21a of the front panel 2A are also called the front body portion 20a and the front extension portion 21a, respectively. The body portion 20b and the extension portion 21b of the rear panel 2B are also called the rear body portion 20b and the rear extension portion 21b, respectively.

The diapers 1, 101 of the first and second embodiments will be described more specifically. As illustrated in FIG. 2 or FIG. 7, the absorbent assembly 3 of the diaper 1, 101 is provided with a liquid permeable topsheet 31, a liquid impermeable or water-repellent backsheet 32, and a liquid retaining absorbent member 33 interposed between both the sheets 31, 32 and is formed into a rectangular shape oblong in direction X. The absorbent member 33 includes an absorbent core formed of an aggregate (may be nonwoven as well) of fibers such as pulp fibers, or an aggregate of fibers and absorbent core having particles of an absorbent polymer retained fibers, and a core wrap sheet (not illustrated) which covers the absorbent core. The absorbent member 33 is formed into a rectangular shape oblong in direction X as well. The absorbent member 33 includes, at both sides in the longitudinal direction, low stiffness portions 33d having no forming material of the absorbent core, or having a basis weight of the forming material being smaller than that of another part. Since the absorbent member 33 is easy to be bent at the low stiffness portion 33d, both the side portions of the absorbent member 33 or the absorbent assembly 3 are more likely to be raised toward wearer's skin when wearing the diaper.

Side cuffs 34, 34 formed of material being liquid-resistant, water-repellent, and permeable are formed at both side portions in the longitudinal direction of the absorbent assembly 3. A side cuff elastic member 35 is arranged at the vicinity of a free end of each side cuff 34 in a stretched state. The side cuff 34 is raised while wearing the diaper 1, 101 owing to contraction of the side cuff elastic member 35 to prevent liquid from leaking outward in the lateral direction from the absorbent assembly 3. The topsheet 31, the backsheet 32, and the absorbent core and the core wrap sheet of the absorbent member 33 may be made of materials respectively being the same which are conventionally used for such kinds of absorbent articles. It is also possible to arrange an external sheet such as nonwoven and a film at the outer side of the absorbent assembly 3 as being overlapped with the backsheet 32.

The front panel 2A of the diaper 1, 101 is rectangular shape oblong in lateral direction in an opened and extended state of the diaper 1, 101 (see FIGs. 2 and 7) and includes the pair of lateral side edge portions 2a, 2a along the diaper longitudinal direction (direction X) and an pair of longitudinal edge portions 2c, 2d (the upper edge portion 2c and the lower edge portion 2d) along the diaper lateral direction (direction Y). Similarly, the rear panel 2B is rectangular shape oblong in the lateral direction in an opened and extended state of the diaper 1 (see FIGs. 2 and 7) and includes the pair of lateral side edge portions 2b, 2b along direction X and an pair of longitudinal edge portions 2c, 2d (the upper edge portion 2c, the lower edge portion 2d) along the diaper lateral direction (direction Y). As illustrated in FIGs. 2 and 7, lengths in the diaper longitudinal direction (direction X) of the front panel 2A and the rear panel 2B are even along the diaper lateral direction (direction Y).

The abovementioned pair of side seals 4, 4 is formed at the diaper 1, 101 by joining the side edge portion 2a (specifically, a joint portion 2a' being a part thereof) of the front panel 2A and the side edge portion 2b (specifically, a joint portion 2b' being a part thereof) of the rear panel 2B. For example, known joining means such as heat sealing, high-frequency sealing, ultrasonic sealing, and adhesive is adopted for the joining. According to the joining, a waist opening 5 and a pair of leg openings 6, 6 are formed as well as the side seals 4, 4.

One end side in the longitudinal direction (a portion overlapped with the front panel 2A) of the absorbent assembly 3 of the diaper 1, 101 is fixed to a center region in direction Y of the front panel 2A with adhesive. The other end side in the longitudinal direction (a portion overlapped with the rear panel 2B) thereof is fixed to a center region in direction Y of the rear panel 2B with adhesive.

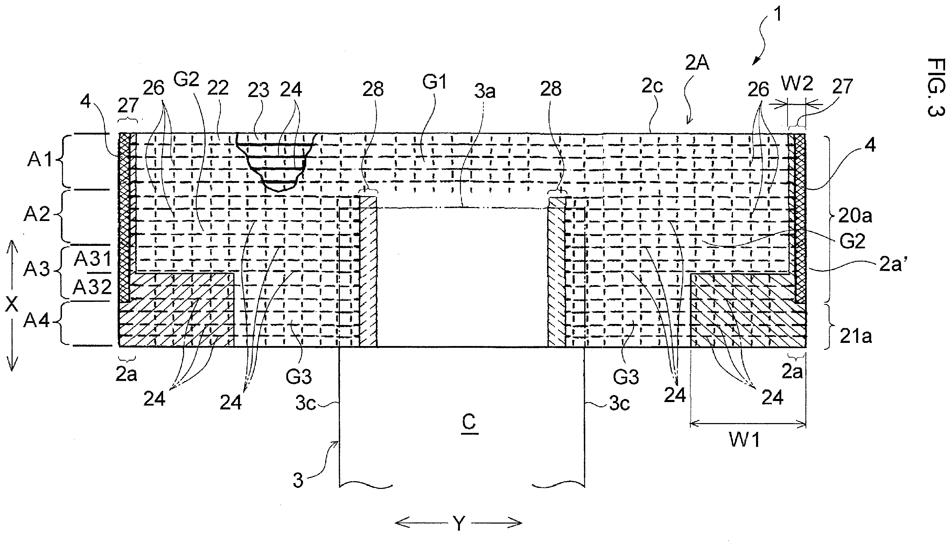

As illustrated in FIGs. 3 and 4 or FIGs. 8 and 9, each of the front panel 2A and the rear panel 2B of the diaper 1, 101 includes an outer layer sheet 22 which forms a diaper outer face, an inner layer sheet 23 arranged at the inner face side of the outer layer sheet 22, and a plurality of continuous-length elastic members 24 arranged between both the sheets 22, 23. A waist stretchable portion G1 and a torso stretchable portion G2 are formed at each of the front body portion 20a and the rear body portion 20b. Further, an extension stretchable portion G3 is formed at each of the front extension portion 21a and the rear extension portion 21b.

At each of the front panel 2A and the rear panel 2B, the waist stretchable portion G1 of the diaper 1, 101 is formed outward of a longitudinal end part 3a, 3b of the absorbent assembly 3 in the longitudinal direction (direction X) of the diaper 1, 101. At each of the front panel 2A and the rear panel 2B, the torso stretchable portion G2 is formed between the waist stretchable portion G1 and the front extension portion 21a or the rear extension portion 21b in direction X. The extension stretchable portion G3 is formed at the front extension portion 21a or the rear extension portion 21b. The torso stretchable portion G2 and the extension stretchable portion G3 are formed at least at positions located outward of both longitudinal edges of the absorbent assembly 3 in the lateral direction (direction Y) of the diaper 1.

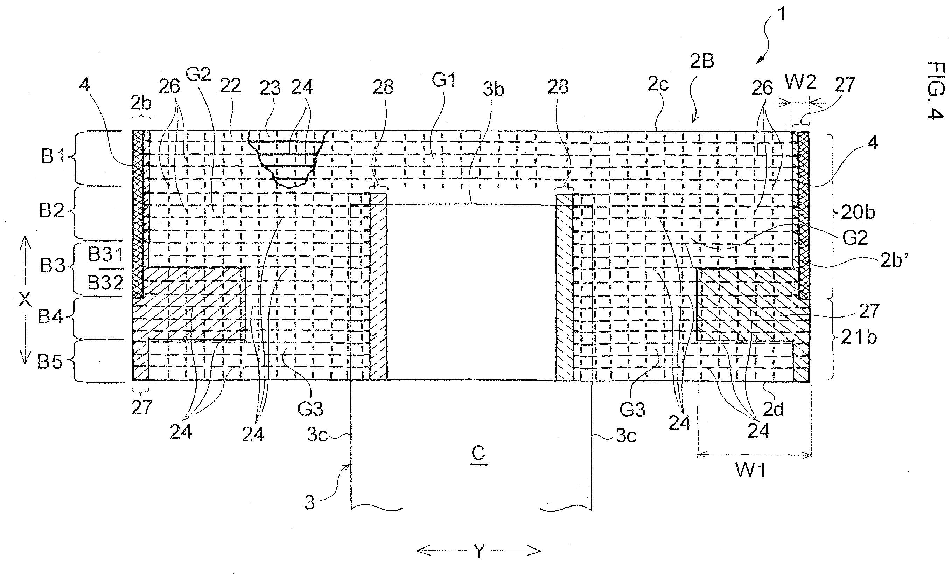

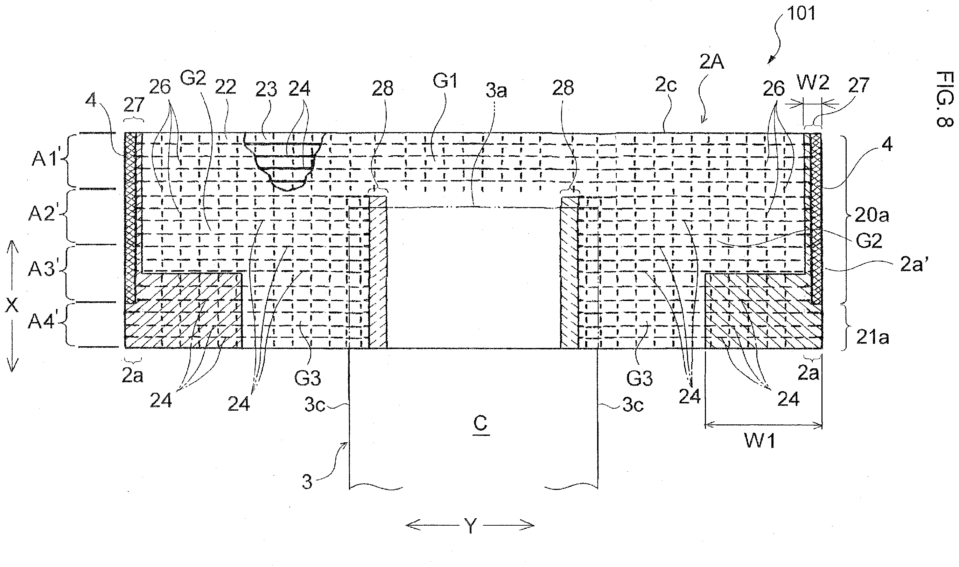

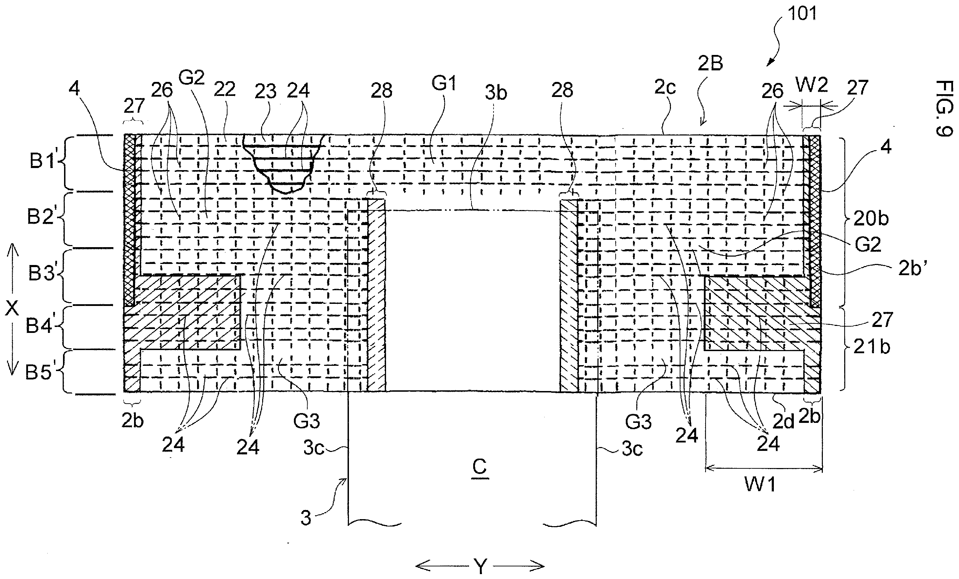

In the diaper 1 illustrated in FIGs. 3 and 4, the waist stretchable portion G1 is configured to provide stretchability over the entire range between side joint regions 27, 27 of the front panel 2A or the rear panel 2B. In contrast, the torso stretchable portion G2 and the extension stretchable portion G3 are configured to be in a state of being separated to the right and left of the diaper 1 so that stretchability is provided to outward of the both side edges of the absorbent assembly 3 and stretchability is not provided to a portion to be overlapped with the absorbent assembly 3, especially, to be overlapped with a center part in the lateral direction of the absorbent assembly 3. The diaper 101 of the second embodiment illustrated in FIGs. 8 and 9 is similar to the above.

The plurality of elastic members 24 is arranged at each of the front body portion 20a, the rear body portion 20b, the front extension portion 21a, and the rear extension portion 21b of the diaper 1, 101, respectively, in a stretched state at intervals in the diaper longitudinal direction as being extended along the diaper lateral direction.

A structure of the diaper 1 of the first embodiment will be described.

In the diaper 1 of the first embodiment, when the rear body portion 20b is sectionalized into three regions B1 to B3 by trisecting the side seal 4 in the article longitudinal direction (direction X) to be a first region B1, a second region B2, and a third region B3 sequentially from a side close to the waist opening as illustrated in FIGs. 2 and 4, the elastic member 24 is arranged at each of the first to third regions B1 to B3 in a stretched state as being extended along the diaper lateral direction. According to the above, the first to third regions B1 to B3 provide stretchability in direction Y, respectively. It is preferable that a plurality of the elastic members 24 is arranged at each of the first to third regions B1 to B3 at intervals in direction X.

Further, when the rear extension portion 21b is sectionalized into an upper rear extension portion B4 and a lower rear extension portion B5 by bisecting the rear extension portion 21b in the article longitudinal direction (direction X), as illustrated in FIGs. 2 and 4, the elastic member 24 is arranged at each of the upper rear extension portion B4 and the lower rear extension portion B5 in a stretched state as being extended along the diaper lateral direction. According to the above, the upper rear extension portion B4 and the lower rear extension portion B5 provide stretchability in direction Y, respectively. It is preferable that a plurality of the elastic members 24 is arranged at each of the upper rear extension portion B4 and the lower rear extension portion B5 at intervals in direction X.

Further, when the front body portion 20a is sectionalized into three regions A1 to A3 by trisecting the side seal 4 in the article longitudinal direction (direction X) to be a first region A1, a second region A2, and a third region A3 sequentially from a side close to the waist opening as illustrated in FIGs. 2 and 3, the elastic member 24 is arranged at each of the first to third region A1 to A3 in a stretched state as being extended along the diaper lateral direction. According to the above, the first to third regions A1 to A3 provide stretchability in direction Y, respectively. It is preferable that a plurality of the elastic members 24 is arranged at each of the first to third regions A1 to A3 at intervals in direction X.

Furthermore, the elastic member 24 is also arranged at the front extension portion 21a of the front panel 2A in a stretched state as being extended along the diaper lateral direction. According to the above, the front extension portion 21a provides stretchability in direction Y as well.

In the diaper 1 of the first embodiment, a length L5 in direction X of the front extension portion 21a is one-half of a length L6 in direction X of the rear extension portion 21b and is the same as a length L61 in direction X of the upper rear extension portion B4. Accordingly, the entire front extension portion 21a is a portion A4 of the front panel 2A corresponding to the upper rear extension portion B4.

Here, in a case that the length L5 in direction X of the front extension portion 21a is longer than the length L61 in direction X of the upper rear extension portion B4, a region in the front extension portion 21a having the length L61 from the body portion 20a is the portion A4 of the front panel 2A corresponding to the upper rear extension portion B4. On the contrary, in a case that the length L5 in direction X of the front extension portion 21a is shorter than the length L61 in direction X of the upper rear extension portion B4, a region having the length L5 shorter than the length L61 is the portion A4 corresponding to the upper rear extension portion B4.

It is preferable that a plurality of the elastic members 24 is also arranged at the portion A4 of the front panel 2A corresponding to the upper rear extension portion B4 at intervals in direction X. In the following, the portion A4 of the front panel 2A corresponding to the upper rear extension portion B4 is called an upper front extension portion A4 as well.

The diaper 1 has stretchability at the upper rear extension portion B4, the lower rear extension portion B5, and the upper front extension portion A4 respectively in the diaper lateral direction (direction X). Contraction stresses in the article lateral direction per unit length in the article longitudinal direction of the upper rear extension portion B4 and the upper front extension portion A4 are larger than a contraction stress of the lower rear extension portion B5 (the contraction stress in the article lateral direction per unit length in the article longitudinal direction), respectively.

That is, relations of the following expressions (2) and (3) are satisfied.

$$\text{Contraction stress of upper rear extension portion B4} > \text{Contraction stress of lower rear extension portion B5} \ldots (2)$$

$$\text{Contraction stress of upper front extension portion A4} > \text{Contraction stress of lower rear extension portion B5} \ldots (3)$$

If large contraction stresses are provided to the front body portion 20a and the rear body portion 20b, especially to the third regions A3, B3 of the front side and the rear side, diaper slip-drop while worn can be prevented.

However, in a case that contraction stresses being large to be capable of preventing slip-drop are provided only to the third regions A3, B3 at the front side and rear side, the regions cause burdens to skin such that a rubber imprint is left with strong contacting to skin.

Meanwhile, when contraction stresses at a certain level being larger than that of the lower rear extension portion B5 are provided to the upper rear extension portion B4 and the upper front extension portion A4 corresponding to the upper rear extension portion B4, slip-drop can be prevented while suppressing a burden to skin by suppressing stresses at the third regions A3, B3.

Further, owing to that the lower rear extension portion B5 provides a contraction stress being smaller than the contraction stress of the upper rear extension portion B4, the lower rear extension portion B5 is softly fitted to a lower part of the largely-curved buttocks. Accordingly, it is possible to prevent protrusion of the buttocks and to provide appearance reliable for leakage. In addition, it is possible to prevent inconvenience such that the rear extension portion 21b is turned up and appearance is worsened with strong contraction of the rear extension portion 21b.

A contraction stress (contraction stress per unit length) at each of the portions A1 to A4 and B1 to B5 can be measured as follows. Contraction stresses at A31, A32, B31, B32, and the like described below may be measured similarly to the above.

The diaper is opened and extended planarly as illustrated in FIG. 2 after the side seals thereof are peeled. The regions A1 to A4 and B1 to B5 of each of the front panel 2A and the rear panel 2B are cut along a straight line parallel to the diaper lateral direction and clipped, so that a reed-shaped or a continuous-length sample of each region over the entire length between both the members 2A, 2B is obtained. At the time of the clipping, the absorbent member is removed in addition to the front panel 2A and the rear panel 2B by cutting the entire diaper including the absorbent assembly 3 and the like. It is also possible that the front panel 2A and the rear panel 2B are cut into the respective regions after removing the absorbent member. In a case that an elastic member exists on a boundary part between regions, cutting is performed at a close part as avoiding the elastic member. Both sides in the longitudinal direction of each clipped region (measurement sample) are sandwiched by a chuck of a tensilon tensile testing machine (Autograph AG-X, manufactured by Shimadzu Corporation). The measurement sample is extended at a speed of 300 mm/min. Here, an inner dimension of the front panel 2A and the rear panel 2B, that is, a length between the side seals of the front panel 2A and the rear panel 2B measured in a state that an external material sheet is not contracted by the elastic member (in other words, in a state that only the front panel 2A and the rear panel 2B are extended without the elastic member arranged) is denoted by "100" (e.g., 350 mm). A contraction stress P in direction Y per unit length in direction X denotes a tensile load (cN) per unit length (10 mm) when the sample is contracted to a length corresponding to "71" (e.g., 250 mm) after being stretched to a length corresponding to "80" (e.g., 280 mm).

The reason of defining a returning force at the length corresponding to "71" against the inner dimension of the front panel 2A and the rear panel 2B denoted by 100 is that a length at the waist of infants being main target wearers of the disposable pull-on diaper 1 of the present embodiment is about 71% against the diaper inner dimension. Here, the length at the waist denotes a mean value of waist lengths measured at a standing position and a sitting position in consideration of variation of circumferential length at the waist when infant posture is varied.

The contraction stress of the third region A3, B3 of each of the front side and the rear side is measured as clipping the entire third region. The contraction stress of the lower half portion A32, B32 of the third region is measured as clipping the lower half portion A32, B32 of the third region.

From a viewpoint to obtain one or two or more effects described above, the contraction stress of the upper rear extension portion B4 is in a range between 1.3 to 3.5 times of the contraction stress of the lower rear extension portion B5 preferably, and in a range of 1.5 to 3 times thereof more preferably. Further, from the similar viewpoint, the contraction stress of the upper rear extension portion B4 is in a rage of 14 to 35 cN preferably, and in a range of 17 to 30 cN more preferably. The contraction stress of the lower rear extension portion B5 is in a range of 1 to 30 cN preferably, and in a range of 5 to 25 cN more preferably.

Further, from the similar viewpoint, the contraction stress of the upper front extension portion A4 is in a range of 1.3 to 3.5 times of the contraction stress of the lower rear extension portion B5 preferably, and in a range of 1.5 to 3 times thereof more preferably. The contraction stress of the upper front extension portion A4 is in a range of 14 to 35 cN preferably, and in a range of 17 to 30 cN more preferably.

Further, for each of the front panel 2A and the rear panel 2B, it is preferable that the first to third regions A1 to A3, B1 to B3 satisfy relations of the following expression (1).

$$\text{Contraction stress of second region} > \text{Contraction stress of third region} > \text{Contraction stress of first region} \ldots (1)$$

That is, regarding the first to third regions A1 to A3 of the front panel 2A, it is preferable that the contraction stress of the second region A2 is the largest, that the contraction stress of the third region A3 is large in the next, and that the contraction stress of the first region A1 is the smallest. Regarding the first to third regions B1 to B3 of the rear panel 2B as well, it is preferable that the contraction stress of the second region B2 is the largest, that the contraction stress of the third region B3 is large in the next, and that the contraction stress of the first region B1 is the smallest.

Further, in the lower half portion A32, B32 of the third region of each of the front panel 2A and the rear panel 2B, it is preferable that the contraction stress thereof in the article lateral direction per unit length in the article longitudinal direction is larger than the contraction stress (contraction stress in the article lateral direction per unit length in the article longitudinal direction) of the first region A1, B1 and is smaller than the contraction stress (contraction stress in the article lateral direction per unit length in the article longitudinal direction) of the second region A2, B2.

That is, regarding the first and second regions A1, A2 and the lower half portion A32 of the third region of the front panel 2A, it is preferable that the contraction stress of the second region A2 is the largest, that the contraction stress of the lower half portion A32 of the third region is large in the next, and that the contraction stress of the first region A1 is the smallest. Regarding the first and second regions B1, B2 and the lower half portion B32 of the third region of the rear panel 2B, it is preferable that the contraction stress of the second region B2 is the largest, that the contraction stress of the lower half portion B32 of the third region is large in the next, and that the contraction stress of the first region B1 is the smallest.

Owing to increasing stresses of the second regions A2, B2 being prone to contact to an iliac section of a wearer while decreasing stresses of the first regions A1, B1 adopted to be worn about the wearer's waist, it is possible to prevent downward slip-drop of a circumferential portion of the waist opening and to lessen a strange feeling due to leakage of excreta at the waist section or protrusion of the vicinity of the infant waist out of the diaper. Further, owing to that at least the stresses of the lower half portions A32, B32 of the third regions A3, B3 are lessened than those of the second regions A2, B2, it is possible to prevent these regions from causing burdens to skin such that a rubber imprint is left with strong contacting to skin.

The iliac section denotes a portion from an iliac crest to an upper front iliac spine of a wearer. For example, JP 2006-61680 A describes about a portion from an iliac crest to an upper front iliac spine.

For having the second regions A2, B2 contacted to the portion from an iliac crest to an upper front iliac spine of a wearer while the diaper 1 is worn, distances k1 and k2 (see FIG. 2) between the diaper center line CL in the longitudinal direction and center positions of the second regions A2, B2 (center positions in the longitudinal direction of the diaper 1) in an opened and extended state of the diaper 1 are in a range of 180 to 230 mm preferably, in a range of 185 to 220 mm more preferably, and in a range of 195 to 215 mm even more preferably, as a diaper for an infant. In a case of a diaper for an adult, the distances k1 and k2 (see FIG. 2) are in a range of 300 to 350 mm preferably, and in a range of 305 to 335 mm more preferably.

In each of the rear panel 2B and the front panel 2A, it is preferable that the contraction stress of the second region B2, A2 is larger than the contraction stress of the third region B3, A3. In other words, in each of the rear panel 2B and the front panel 2A, it is preferable that the contraction stress of the third region B3, A3 is smaller than the contraction stress of the second region B2, A2. The contraction stress of the second region B2, A2 is in a range of 1.3 to 2.5 times of the contraction stress of the third region B3, A3 preferably, and in a range of 1.3 to 2 times thereof more preferably.

Further, in each of the rear panel 2B and the front panel 2A, it is preferable that the contraction stress of the second region B2, A2 is larger than the contraction stress of the lower half portion B32, A32 of the third region. In other words, in each of the rear panel 2B and the front panel 2A, it is preferable that the contraction stress of the lower half portion B32, A32 of the third region is smaller than the contraction stress of the second region B2, A2. The contraction stress of the second region B2, A2 is in a range of 1.3 to 2.5 times of the contraction stress of the lower half portion B32, A32 of the third region preferably, and in a range of 1.3 to 2 times thereof more preferably.

Further, in each of the rear panel 2B and the front panel 2A, the contraction stress of the third region B3, A3 is in a range of more than 1 time to 3.5 times or less of the contraction stress of the first region B1, A1 preferably, and in a range of more than 1 time and 3.0 times or less thereof more preferably.

Further, in each of the rear panel 2B and the front panel 2A, the contraction stress of the lower half portion B32, A32 of the third region is in a range of 1.2 to 3.5 times of the contraction stress of the first region B1, A1 preferably, and in a range of 1.2 to 3.0 times more preferably.

Further, in each of the rear panel 2B and the front panel 2A, the contraction stress of the second region B2, A2 is in a range of 14 to 50 cN preferably, and in a range of 20 to 50 cN more preferably.

Further, in each of the rear panel 2B and the front panel 2A, the contraction stress of the third region B3, A3 is in a range of 14 to 35 cN preferably, and in a range of 17 to 35 cN more preferably.

Further, in each of the rear panel 2B and the front panel 2A, the contraction stress of the first region B1, A1 is in a range of 1 to 30 cN preferably, and in a range of 5 to 25 cN more preferably.

From a viewpoint to prevent slippage with the contraction stresses of the third regions A3, B3, the upper rear extension portion B4, and the upper front extension portion A4 described above while preventing the third regions A3, B3, especially the lower half portions A32, B32 thereof from being strongly contacted to skin, it is preferable that the contraction stress of the upper rear extension portion B4 is smaller than the contraction stress of the second region B2 of the rear panel 2B. Further, from the similar viewpoint, it is preferable that the contraction stress of the upper front extension portion A4 is smaller than the contraction stress of the second region A2 of the front panel 2A. It is more preferable that both of the upper rear extension portion B4 and the upper front extension portion A4 satisfy the above conditions.

In each of the third regions A3, B3, at least one elastic member 24 is arranged respectively at the upper half portion A31, B31 and the lower half portion A32, B32 preferably, and a plurality of elastic members 24 is arranged at intervals in direction X more preferably.

As being located at the vicinity of the crotch portion of the diaper having increased weight with urination, the upper rear extension portion B4 and the lower half portion B32 of the third region B3 of the rear panel 2B are portions where rubber imprints are easy to be left as a result of large pressure under urination.

Owing to that the contraction stress of the upper rear extension portion B4 and the contraction stress of the lower half portion B32 of the third region B3 of the rear panel 2B are equalized, pressure under urination applied to the upper rear extension portion B4 and the lower half portion B32 of the third region B3 of the rear panel 2B is evenly dispersed. Accordingly, since the pressure can be prevented from being concentrated on any portion, it is preferable that rubber imprints can be effectively reduced while preventing slip-drop.

From the similar viewpoint, it is preferable that the contraction stress of the upper front extension portion A4 and the contraction stress of the lower half portion A32 of the third region A3 of the front panel 2A are equalized.

The contraction stress of the lower half portion B32 of the third region B3 of the rear panel 2B is in a range of 0.3 to 2.5 times of the contraction stress of the upper rear extension portion B4 preferably, and in a range of 0.5 to 2 times thereof more preferably. Here, the equalization denotes a case that the ratio is in a range of 0.8 to 1.2 times.

Further, the contraction stress of the second region B2 of the rear panel 2B is in a range of 1.3 to 2.5 times of the contraction stress of the upper rear extension portion B4 preferably, and in a range of 1.3 to 2 times thereof more preferably.

The contraction stress of the lower half portion B32 of the third region B3 of the rear panel 2B is in a range of 14 to 35 cN preferably, and in a range of 17 to 35 cN more preferably.

From a viewpoint to further improve diaper stretchability and further decrease occurrence of rubber imprints while preventing slip-drop, it is preferable that the contraction stress of the upper half portion B31 of the third region B3 of the rear panel 2B is equal to or smaller than the contraction stress of the lower half portion B32.

Here, from a viewpoint to prevent slip-drop, it is preferable that the contraction stress of the third region B3 is larger than the contraction stress of the first region B1.

The contraction stress of the lower half portion A32 of the third region A3 of the front panel 2A is in a range of 0.3 to 2.5 times of the contraction stress of the upper front extension portion A4 preferably, and in a range of 0.5 to 2.0 times thereof more preferably. Here, the equalization denotes a case that the ratio is in a range of 0.8 to 1.2 times.

Further, the contraction stress of the second region A2 of the front panel 2A is in a range of 1.3 to 2.5 times of the contraction stress of the upper front extension portion A4 preferably, and in a range of 1.3 to 2 times thereof more preferably.

The contraction stress of the lower half portion A32 of the third region A3 of the front panel 2A is in a range of 14 to 35 cN preferably, and in a range of 17 to 35 cN more preferably.

From a viewpoint to further improve diaper stretchability and further decrease occurrence of rubber imprints while preventing slip-drop, it is preferable that the contraction stress of the upper half portion A31 of the third region A3 of the front panel 2A is equal to or smaller than the contraction stress of the lower half portion A32.

Here, from a viewpoint to prevent slip-drop, it is preferable that the contraction stress of the third region A3 is larger than the contraction stress of the first region A1.

The method to differentiate a contraction stress of a region of each of the front panel 2A and the rear panel 2B from that of another region thereof is not specifically limited as long as being capable of differentiating contraction stresses. Examples thereof include (i) a method to differentiate stretch rates of elastic members between a region having a large contraction stress and a region having a small contraction stress when the elastic members are fixed to the sheet 22 and/or the sheet 23, (ii) a method to differentiate thicknesses of elastic members arranged between a region having a large contraction stress and a region having a small contraction stress, (iii) a method to differentiate materials of elastic members arranged between a region having a large contraction stress and a region having a small contraction stress, (iv) a method to differentiate arrangement intervals of elastic members between a region having a large contraction stress and a region having a small contraction stress, and (v) a method in which two or more of the above are combined.

A structure of the diaper 101 of the second embodiment will be described.

In the diaper 101 of the second embodiment, the length L6 in direction X of the rear extension portion 21b is longer than the length L5 in direction X of the front extension portion 21a (hereinafter, the front extension portion 21a is called the front extension portion A4' as well). Here, the rear extension portion 21b includes a rear corresponding portion B4' which corresponds to the front extension portion 21 (A4') and a lower extension portion B5' which is extended from the rear corresponding portion B4'toward the crotch portion C.

The rear corresponding portion B4' is located in the rear extension portion 21b on the side of the crotch portion C from the rear body portion 20b. The distance from the rear body portion 20b to the rear corresponding portion B4' is equal to zero or larger and is equal to the length L5 in direction X of the front extension portion A4' or smaller.

In contrast, when the length L5 in direction X of the front extension portion A4' is equal to the length L6 in direction X of the rear extension portion 21b, the entire rear extension portion 21b is to be the rear corresponding portion B4'.

Further, when the front body portion 20a is sectionalized into three regions A1' to A3' by trisecting the side seal 4 in the article longitudinal direction (direction X) to be a first region A1', a second region A2', and a third region A3' sequentially from a side close to the waist opening as illustrated in FIGs. 7 and 8, the elastic member 24 is arranged at each of the first to third region A1' to A3' in a stretched state as being extended along the diaper lateral direction. According to the above, the first to third regions A1' to A3' provide stretchability in direction Y, respectively.

Furthermore, the elastic member 24 is also arranged at the front extension portion A4' of the front panel 2A in a stretched state as being extended along the diaper lateral direction. According to the above, the front extension portion A4' provides stretchability in direction Y as well.

In the front panel 2A, it is preferable that a plurality of the elastic members 24 is arranged at each of the first to third regions A1' to A3' and front extension portion 21a (A4') at intervals in direction X. In a case that a width of each of the first to third regions A1' to A3' is set to be about 40 mm, the number of the elastic members arranged at intervals in direction X for each of the regions A1' to A3' is in a range of 2 to 80 preferably, and in a range of 2 to 20 more preferably. For each of the front third region A3' and the front extension portion 21a (A4'), the number is in a range of 2 to 80 preferably, and in a range of 2 to 20 more preferably.

The numeral range of the number of the elastic members to be arranged in the front panel 2A varies based on the widths of the respective regions A1' to A3'. In a case that the widths of the respective regions A1' to A3' are larger (or smaller) than 40 mm, the maximum number of the numeral range becomes to a value obtained by multiplying the abovementioned maximum number by a value obtained by dividing the width of the region by 40. For example, in a case that a width of each of the regions A1' to A3' is set to be 80 mm, the number of the elastic members arranged in each of the regions A1' to A3' is in a range of 2 to 160 preferably, and in a range 2 to 40 more preferably. For each of the front third region and the front extension portion 21a (A4'), the number is in a range of 2 to 160 preferably, and in a range of 2 to 40 more preferably.

Further, when the rear body portion 20b is sectionalized into three regions B'1 to B3' by trisecting the side seal 4 in the article longitudinal direction (direction X) to be a first region B1', a second region B2', and a third region B3' sequentially from a side close to the waist opening as illustrated in FIGs. 7 and 9, the elastic member 24 is arranged at each of the first to third regions B1' to B3' in a stretched state as being extended along the diaper lateral direction. According to the above, the first to third regions B1' to B3' provide stretchability in direction Y, respectively.

Further, the elastic member 24 is arranged at each of the rear corresponding portion B4' and the lower extension portion B5' of the rear extension portion 21b in a stretched state as being extended along the diaper lateral direction. According to the above, the rear corresponding portion B4' and the lower extension portion B5' provide stretchability in direction Y, respectively.

In the rear panel 2B, it is preferable that a plurality of the elastic members 24 is arranged at each of the first to third regions B1' to B3', the rear corresponding portion B4', and the lower extension portion B5' at intervals in direction X. In a case that a width of each of the first to third regions B1' to B3' is set to be about 40 mm, the number of the elastic members arranged at intervals in direction X for each of the regions B1' to B3' is in a range of 2 to 80 preferably, and in a range of 2 to 20 more preferably. For each of the rear third region B3' and the rear corresponding portions B4', the number is in a range of 2 to 80 preferably, and in a range of 2 to 20 more preferably. For the lower extension portion B5', the number is in a range of 2 to 80 preferably, and in a range of 2 to 20 more preferably.

The numeral range of the number of the elastic members to be arranged in the front panel 2B varies based on the widths of the respective regions B1' to B3'. In a case that the widths of the respective regions B1' to B3' are larger (or smaller) than 40 mm, the maximum number of the numeral range becomes to a value obtained by multiplying the abovementioned maximum number by a value obtained by dividing the width of the region by 40. For example, in a case that a width of each of the regions B1' to B3' is set to be 80 mm, the number of the elastic members arranged in each of the regions B1' to B3' is in a range of 2 to 160 preferably, and in a range 2 to 40 more preferably. For each of the rear third region B3' and the rear corresponding portion B4', the number is in a range of 2 to 160 preferably, and in a range of 2 to 40 more preferably. For the lower extension portion B5', the number is in a range of 2 to 160 preferably, and in a range of 2 to 40 more preferably.

Under definitions that $P_{A1'}\sim P_{A4'}$ denote contraction stresses in the article lateral direction of the first to third regions A1' to A3' and the front extension portion 21a (A4') of the front panel 2A per unit length in the article longitudinal direction, that $Q_{A1'}\sim Q_{A4'}$ denote contraction stresses in the article lateral direction per single elastic member, that $P_{B1'}\sim P_{B4'}$ denote contraction stresses in the article lateral direction of the first to third regions B1' to B3' and the rear corresponding portion B4' of the rear panel 2B per unit length in the article longitudinal direction, and that $Q_{B1'}\sim Q_{B4'}$ denote contraction stresses in the article lateral direction per single elastic member, the diaper 101 satisfies the following relational expressions (11) to (14).

$$\text{Ratio } (P_{A3'}/P_{A1'}) > \text{Ratio } (Q_{A3'}/Q_{A1'}), \text{ Ratio } (P_{A3'}/P_{A1'}) > 1 \ldots (11)$$

$$\text{Ratio } (P_{B3'}/P_{B1'}) > \text{Ratio } (Q_{B3'}/Q_{B1'}), \text{ Ratio } (P_{B3'}/P_{B1'}) > 1 \ldots (12)$$

$$\text{Ratio } (P_{A4'}/P_{A1'}) > \text{Ratio } (Q_{A4'}/Q_{A1'}), \text{ Ratio } (P_{A4'}/P_{A1'}) > 1 \ldots (13)$$

$$\text{Ratio } (P_{B4'}/P_{B1'}) > \text{Ratio } (Q_{B4'}/Q_{B1'}), \text{ Ratio } (P_{B4'}/P_{B1'}) > 1 \ldots (14)$$

The relational expressions (11) and (12) indicate, in each of the front panel 2A and the rear panel 2B, that the third region A3', B3' has a larger contraction stress in direction Y per unit length in direction X than the first region A1', B1' and that the difference between the contraction stresses in direction Y per single elastic member 24 of both the regions is not that large.

The relational expressions (13) and (14) indicate, in each of the front panel 2A and the rear panel 2B, that the fourth region (the front extension portion A4', the rear corresponding portion B4') has a larger contraction stress in direction Y per unit length in direction X than the first region A1', B1' and that the difference between contraction stresses in direction Y per single elastic member 24 of both the regions is not that large.

A pull-on absorbent article in which the external material is divided into the front panel 2A and the rear panel 2B is prone to cause slip-down while worn. Here, when contraction stresses in the article lateral direction of the rear panel and the front panel are enlarged specifically at a range in the vicinity of a lower end part of the side seal (an end part at the crotch portion side), diaper slip-drop can be effectively prevented.

However, in the vicinity of the lower end part of the side seal (the end part at the crotch portion side), a rubber imprint is prone to be left at wearer's skin and a burden is prone to be laid on skin. A cross-sectional shape of a wearer's body is close to oval at a portion to which the vicinity of the lower end part of the side seal (the end part at the crotch portion side) is contacted. This is considered to be the reason why a rubber imprint is prone to be left compared to another portion.

On the contrary, in the diaper 101, the extension portions A4', 21b are arranged at both the front panel 2A and the rear panel 2B, respectively. Here, regarding the contraction stress per unit length, the contraction stress of each of the third regions A3', B3', front extension portion A4', and the rear corresponding portion B4' which are located at the vicinity of the lower end part of the side seal is set larger than that of the first region A1', B1'. Further, regarding the contraction stress per single elastic member, the contraction stresses of the third regions A3', B3', the front extension portion A4', and the rear corresponding portion B4' are decreased. According to the above, it is possible to obtain a diaper (pull-on absorbent article) having a small burden to skin with pressing imprints of elastic members less likely to be left as excellently preventing slip-drop while worn.

Further, owing to decreasing the contraction stress per unit length of the first region A1', B1' adapted to be worn about the waist, downward slip-drop of the circumferential portion of the waist opening can be effectively prevented. In addition, a burden to skin about the waist can be prevented while preventing a pressing imprint from being left about the wearer's waist. Thus, owing to decreasing the contraction stress of the first region per unit length and further to appropriately increasing the contraction stress at the vicinity of the lower end part of the side seal (the third region, the fourth region) per unit length compared to the first region, it is possible to prevent both of slip-drop from the periphery of the waist and slip-drop from the vicinity of the side seal lower end part. Further, owing to lessening difference between the contraction stresses of the first region and the third region per single elastic member, it is possible at the third and fourth regions as well to reduce pressing imprint of the elastic members to a similar level to that of the first region which has small contraction stress. From the viewpoints of the above, each of the ratio ($Q_{A3'}/Q_{A1'}$), the ratio ($Q_{A4'}/Q_{A1'}$), the ratio ($Q_{B3'}/Q_{B1'}$), and the ratio ($Q_{B4'}/Q_{B1'}$) may be set to one.

Further, it is preferable that the diaper 101 satisfies the following relational expressions (15) and (16).

$$P_{A3'} = P_{A4'}, \ Q_{A3'} = Q_{A4'} \quad \dots \quad (15)$$

$$P_{B3'} = P_{B4'}, \ Q_{B3'} = Q_{B4'} \quad \dots \quad (16)$$

The relational expression (15) indicates that the contraction stresses per unit length in direction X are the same at the third region A3' and the front extension portion A4' of the front panel 2A and that the contraction stresses per single elastic member 24 are also the same at the third region A3' and the front extension portion A4' of the front panel 2A.

The relational expression (16) indicates that the contraction stresses in direction Y per unit length in direction X are the same at the third region B3' and the rear corresponding portion B4' of the rear panel 2B and that the contraction stresses in direction Y per single elastic member 24 are the same at the third region B3' and the rear corresponding portion B4' of the rear panel 2B.

Owing to satisfying the relational expressions (15) and (16), it is possible to evenly decrease stresses at the vicinity of the side seal lower end part where a rubber imprint is prone to be left without being concentrated at either the third region or the fourth region. Accordingly, rubber imprints can be totally reduced at the vicinity of the side seal end part.

Here, being equal of the contraction stresses in direction Y per unit length in direction X also includes a case that an absolute value of the difference between the contraction stresses in direction Y of both the above per unit length in direction X is 2.5 cN or less in addition to a case of being completely equal. Further, being equal of the contraction stresses in direction Y per single elastic member 24 also includes a case that an absolute value of the difference between the contraction stresses in direction Y of both the above per single elastic member 24 is 2.5 cN or less in addition to a case of being completely equal.

A contraction stress of each of the regions A1' to A4' and B1' to B5' is measured as follows.

[Measurement sample]

The diaper is opened and extended planarly as illustrated in FIG. 7 after the side seals thereof are unstuck. The regions A1' to A4' and B1' to B5' of each of the front panel 2A and the rear panel 2B are cut along a straight line parallel to the diaper lateral direction and clipped, so that a reed-shaped or a continuous-length sample of each region over the entire length between both side edge portions of the members 2A, 2B is obtained. At the time of the clipping, the absorbent member is removed in addition to the front panel 2A and the rear panel 2B by cutting the entire diaper including the absorbent assembly 3 and the like. It is also possible that the front panel 2A and the rear panel 2B are cut into the respective regions after removing the absorbent member. In a case that an elastic member exists on a boundary part between regions, cutting is performed at a close part as avoiding the elastic member.

[Measurement of contraction stress P in direction Y per unit length in direction X]

Both sides of the sample are sandwiched by a chuck of a tensile testing machine (Autograph AG-X, manufactured by Shimadzu Corporation). The sample is extended at a speed of 300 mm/min. Here, an inner dimension of the front panel 2A and the rear panel 2B, that is, a length between the side seals of the front panel 2A and the rear panel 2B measured in a state that an external material sheet is not contracted by the elastic member (in other words, in a state that only the front panel 2A and the rear panel 2B are extended without the elastic member arranged) is denoted by "100" (e.g., 350 mm). A contraction stress P in direction Y per unit length in direction X denotes a tensile load (cN) per unit length (10 mm) when the sample is contracted to a length corresponding to "71" (e.g., 250 mm) after being stretched to a length corresponding to "80" (e.g., 280 mm). The reason of defining a returning force at the length corresponding to "71" against the inner dimension of the front panel 2A and the rear panel 2B denoted by 100 is that a length at the waist of infants being main target wearers of the disposable pull-on diaper 101 of the present embodiment is about 71% against the diaper inner dimension. Here, the length at the waist denotes a mean value of waist lengths measured at a standing position and a sitting position in consideration of variation of circumferential length at the waist when infant posture is varied.

[Measurement of contraction stress Q in direction Y per single elastic member]

Both sides of the sample are sandwiched by a chuck of a tensile testing machine (Autograph AG-X, manufactured by Shimadzu Corporation). The sample is extended at a speed of 300 mm/min. Here, an inner dimension of the front panel 2A and the rear panel 2B, that is, a length between the side seals of the front panel 2A and the rear panel 2B measured in a state that an external material sheet is not contracted by the elastic member (in other words, in a state that only the front panel 2A and the rear panel 2B are extended without the elastic member arranged) is denoted by "100" (e.g., 350 mm). A contraction stress Q in direction Y per single elastic member denotes a value obtained by dividing the contraction stress when the sample is contracted to a length corresponding to "71" (e.g., 250 mm) after being stretched to a length corresponding to "80" (e.g., 280 mm) by the number of elastic members fixed in the measured sample. The reason of defining a returning force at the length corresponding to "71" against the inner dimension of the front panel 2A and the rear panel 2B denoted by 100 is that a length at the waist of infants being main target wearers of the disposable pull-on diaper 101 of the present embodiment is about 71% against the diaper inner dimension. Here, the length at the waist denotes a mean value of waist lengths measured at a standing position and a sitting position in consideration of variation of circumferential length at the waist when infant posture is varied.

From a viewpoint of preventing slip-drop, pressing imprint of elastic members, and burdens to skin or a viewpoint of reliably obtaining one or two or more effects described below, each of the ratio ($P_{A3'}/P_{A1'}$), the ratio ($P_{B3'}/P_{B1'}$), the ratio ($P_{A4'}/P_{A1'}$), and the ratio ($P_{B4'}/P_{B1'}$) is in a range of 1.5 to 7.0 preferably, in a range of 1.6 to 5.0 more preferably, and in a range of 1.7 to 3.5 even more preferably.

Further, from the similar viewpoints, each of the ratio ($Q_{A3'}/Q_{A1'}$), the ratio ($Q_{B3'}/Q_{B1'}$), the ratio ($Q_{A4'}/Q_{A1'}$), and the ratio ($Q_{B4'}/Q_{B1'}$) is in a range of 0.3 to 2.2 preferably, in a range of 0.5 to 1.8 more preferably, and in a range of 0.7 to 1.4 even more preferably.

Further, it is preferable that the diaper 101 satisfies the following relational expressions (17) and (18).

$$4\,cN < Q_{A3'} < 23\,cN \quad \dots \quad (17)$$

$$4\,cN < Q_{B3'} < 23\,cN \quad \dots \quad (18)$$

The relational expressions (17) and (18) indicate that in both of the front panel 2A and the rear panel 2B, the contraction stresses in direction Y of the third regions A3', B3' per single elastic member 24 are larger than 4 cN and smaller than 23 cN.

Owing to satisfying the relational expressions (17) and (18), a pressing imprint (rubber imprint) of an elastic member is less likely to be left and a burden to skin is small even with usage in the season when an infant is prone to feel sweaty at high temperature or under circumstances in which a rubber imprint is prone to be left such that wearing time is long. In addition to being a burden to skin due to pressing, such a rubber imprint may cause redness and rashes, and in a severe case, may cause hurts. Even when a rash or a hurt does not appear, mothers may feel insecure about development thereof or child's strange feelings due to stresses. If the contraction stress is excessively decreased to prevent rubber imprints, diaper slippage occurs and excreta leaks through clearance caused by the slippage and an absorbent member at the crotch portion dangles. In such a case, motion of an infant is to be disturbed. Owing to satisfying the relational expressions (17) and (18), it is possible to solve both the problems being rubber print prevention and slip-drop prevention.

Each of $Q_{A3'}$ and $Q_{B3'}$ is larger than 4 cN and smaller than 20 cN preferably, and larger than 4 cN and smaller than 15 cN more preferably.

From a viewpoint of preventing slip-drop, pressing imprint of elastic members, and burdens to skin or a viewpoint of obtaining one or two or more effects described below, the contraction stress $P_{B3'}$ in direction Y of the rear third region B3' per unit length in direction X is in a range of 14 to 40 cN preferably, and in a range of 17 to 35 cN more preferably. Further, the contraction stress $P_{A3'}$ in direction Y of the front third region A3' per unit length in direction X is in a range of 14 to 40 cN preferably, and in a range of 17 to 35 cN more preferably. From the similar viewpoints, the contraction stress $P_{B4'}$ in direction Y of the rear corresponding portion B4' per unit length in direction X is in a range of 14 to 40 cN preferably, and in a range of 17 to 35 cN more preferably. Further, the contraction stress $P_{B5'}$ in direction Y of the lower extension portion B5' per unit length in direction X is in a range of 5 to 30 cN preferably, and in a range of 5 to 25 cN more preferably. Similarly, the contraction stress $P_{A4'}$ in direction Y of the front extension portion A4' per unit length in direction X is in a range of 14 to 40 cN preferably, and in a range of 17 to 35 cN more preferably.

Further from viewpoints to effectively prevent downward slip-drop of the circumferential portion of the waist opening and to prevent a burden to skin about the waist while preventing a pressing imprint from being left about the wearer's waist, in each of the rear panel 2B and the front panel 2A, the contraction stresses $P_{B1'}$, $P_{A1'}$ in direction Y of the first regions B1', A1' per unit length in direction X are respectively in a range of 5 to 30 cN preferably, and in a range of 5 to 25 cN more preferably.

From a viewpoint of preventing slip-drop, pressing imprint of elastic members, and burdens to skin or a viewpoint of obtaining one or two or more effects described below, the contraction stress $Q_{B4'}$ in direction Y of the rear corresponding portion B4' per single elastic member is in a range of 4 to 23 cN preferably, and in a range of 4 to 20 cN more preferably. Further, the contraction stress $Q_{A4'}$ in direction Y of the front extension portion A4' is in a range of 4 to 23 cN preferably, and in a range of 4 to 20 cN more preferably.

It is preferable that the diaper 101 satisfies the following relational expressions (19) and (20).

$$P_{A2'} > P_{A3'}, P_{B2'} > P_{B3'} \quad \ldots \quad (19)$$

$$P_{A2'} > P_{A4'}, P_{B2'} > P_{B4'} \quad \ldots \quad (20)$$

The relational expression (19) indicates that the contraction stresses of the third regions A3', B3' are smaller than the contraction stresses of the second regions A2' and B2' in each of the front panel 2A and the rear panel 2B regarding the contraction stresses in direction Y per unit length in direction X.

The relational expression (20) indicates that the contraction stresses of the front extension portion A4' and the rear corresponding portion B4' are smaller than the contraction stresses of the second regions A2' and B2' regarding the contraction stresses in direction Y per unit length in direction X.

Owing to that the contraction stresses per unit length of the second regions A2', B2' which are prone to contact to an iliac section of a wearer are increased and that the contraction stresses per unit length of the first regions of the circumferential portion of the waist opening are decreased to be lower than those of the second regions, downward slip-drop of the circumferential portion of the waist opening can be effectively prevented. Further, owing to that the contraction stresses per unit length of the third regions A3', B3' and the front extension portion A4' and the rear corresponding portion B4' which are located below the iliac section of a wearer and are relatively prone to receive a rubber imprint compared to other regions as having a cross-sectional shape of the body being closer to oval than the iliac section are set smaller than those of the second regions A2', B2', it is possible to prevent phenomenon more effectively such that those regions strongly contact to skin and cause a burden to skin with an imprint left thereat.

Here, each of the ratio ($P_{A2'}/P_{A3'}$), the ratio ($P_{B2'}/P_{B3'}$), the ratio ($P_{A2'}/P_{A4'}$), and the ratio ($P_{B2'}/P_{B4'}$) is larger than 1 and equal to or smaller than 4 preferably, larger than 1 and equal to or smaller than 3.5 more preferably, and larger than 1 and equal to or smaller than 2.5 even more preferably.

Further, from viewpoints to effectively prevent downward slip-drop of the circumferential portion of the waist opening, to prevent a pressing imprint from being left about the iliac section, and to prevent a burden to skin about the iliac section, in each of the rear panel 2B and the front panel 2A, the contraction stresses $P_{B2'}$, $P_{A2'}$ in direction Y of the second regions B2', A2' per unit length in direction X are in a range of 14 to 50 cN preferably, and in a range of 20 to 50 cN more preferably. Further, in each of the rear panel 2B and the front panel 2A, the contraction stresses $Q_{B2'}$, $Q_{A2'}$ in direction Y of the second region B2', A2' per single elastic member are in a range of 4 to 23 cN preferably, and in a range of 4 to 20 cN more preferably.

The iliac section denotes a portion from an iliac crest to an upper front iliac spine of a wearer. For example, JP 2006-61680 A describes about a portion from an iliac crest to an upper front iliac spine.

For having the second regions A2', B2' contacted to the portion from an iliac crest to an upper front iliac spine of a wearer while the diaper 101 is worn, distances k1 and k2 (see FIG. 7) between the diaper center line CL in the longitudinal direction and center positions of the second regions A2', B2' (center positions in the longitudinal direction of the diaper 101) in an opened and extended state of the diaper 101 is in a range of 180 to 230 mm preferably, in a range of 185 to 220 mm more preferably, and in a range of 195 to 215 mm even more preferably, as a diaper for an infant. In a case of a diaper for an adult, the distances k1 and k2 (see FIG. 7) is in a range of 300 to 350 mm preferably, and in a range of 305 to 335 mm more preferably.

Further, it is preferable that the diaper 101 satisfies the following relational expressions (22) to (25).

$$\text{Ratio } (P_{A2'}/P_{A3'}) > \text{Ratio } (Q_{A2'}/Q_{A3'}), \text{ Ratio } (P_{A2'}/P_{A3'}) > 1 \dots (22)$$

$$\text{Ratio } (P_{B2'}/P_{B3'}) > \text{Ratio } (Q_{B2'}/Q_{B3'}), \text{ Ratio } (P_{B2'}/P_{B3'}) > 1 \dots (23)$$

$$\text{Ratio } (P_{A2'}/P_{A4'}) > \text{Ratio } (Q_{A2'}/Q_{A4'}), \text{ Ratio } (P_{A2'}/P_{A4'}) > 1 \dots (24)$$

$$\text{Ratio } (P_{B2'}/P_{B4'}) > \text{Ratio } (Q_{B2'}/Q_{B4'}), \text{ Ratio } (P_{B2'}/P_{B4'}) > 1 \dots (25)$$

The second region corresponds to the iliac section. It is effective to increase the contraction stress of the second region while reducing the contraction stress of the first region for preventing diaper slippage from the circumferential portion of the waist opening. In particular, the circumferential portion of the waist opening has large variation of circumferential length due to motion such as standing and sitting and is prone to cause diaper slippage due to motion similarly to or more than the periphery of the side seal lower end portion. Owing to satisfying the relational expressions (22) to (25) and (11) to (14), it is possible to prevent both diaper slippage from the waist and slippage of the periphery of the side seal lower end portion. Further, it is possible to reduce rubber imprints over the entire range about the waist by decreasing the stress per unit number at the iliac section as being similar to the waist section. From the viewpoints of the above, each of the ratio $(Q_{A2'}/Q_{A3'})$, the ratio $(Q_{B2'}/Q_{B3'})$, the ratio $(Q_{A2'}/Q_{A4'})$, and the ratio $(Q_{B2'}/Q_{B4'})$ may be set to one.

Here, as described above, being equal of the contraction stresses in direction Y per single elastic member 24 also includes a case that an absolute value of the difference between the contraction stresses in direction Y of both the above per single elastic member 24 is 2.5 cN or less in addition to a case of being completely equal.

Under definitions that $P_{B5'}$ denotes the contraction stress in direction Y of the lower extension portion B5' per unit length in direction X and $Q_{B5'}$ denotes the contraction stress in direction Y per single elastic member, the diaper 101 satisfies the following relational expression (21).

$$\text{Ratio } (P_{B4'}/P_{B5'}) > \text{Ratio } (Q_{B4'}/Q_{B5'}), \text{ Ratio } (P_{B4'}/P_{B5'}) > 1 \dots (21)$$

The relational expression (21) indicates that the contraction stress in direction Y of the lower extension portion B5' is smaller than that of the rear corresponding portion B4' and that the difference between the contraction stresses in direction Y per single elastic member of the rear corresponding portion B4' and the lower extension portion B5' is not that large as being smaller than the difference between the contraction stresses per unit length in direction X of the rear corresponding portion B4' and the lower extension portion B5'. Owing to that the contraction stress in direction Y per unit length in direction X of the lower extension portion B5' is smaller than that of the rear corresponding portion B4', the entire range of the extension portion is more likely to be fitted to the buttocks especially at the largely-curved lower side of the buttocks. Further, since the contraction stress of the lower extension portion B5' is small, the width in direction Y remains large when the lower extension portion B5' is contracted. As a result, area to cover the buttocks becomes large, so that protrusion of the buttocks from the diaper can be prevented and an uneasy feeling for leakage and a strange feeling can be reduced. Regarding the effects obtained by the large width in direction Y while the extension portion is contracted, the effect to further prevent protrusion of the buttocks can be obtained by setting a stretch rate when fixing the elastic members to the lower extension portion B5' to be smaller than a stretch rate when fixing the elastic members to the rear corresponding portion B4'. Further, setting the difference between the contraction stresses per single elastic member to be smaller than the difference between the contraction stresses per unit length leads to that rubber imprints can be prevented from being left over the entire range of the extension portion while preventing rubber imprints at the rear corresponding portion B4' owing to that a pressing imprint of the elastic member at the rear corresponding portion B4' where a rubber print is prone to be left is formed close to that of the lower extension portion having a structure in which a rubber imprint is less likely to be left with a small contraction stress per unit length and a small contraction stress per single elastic member. From the viewpoints of the above, the ratio ($Q_{B4'}/Q_{B5'}$) may be set to one.

As a method to set the contraction stress $P_{B5'}$ of the lower extension portion B5' to be smaller than the contraction stress $P_{B4'}$ of the rear corresponding portion B4', it is preferable that an arrangement interval in the article longitudinal direction of the elastic members at the rear corresponding portion B4' is set to be larger than an arrangement interval in the article longitudinal direction of the elastic members at the lower extension portion B5'. In addition to the abovementioned effects obtained by decreasing the contraction stress with the enlarged arrangement interval, the above is preferable because of that bending stiffness in direction X of the sheet member including the elastic members of the lower extension portion can be decreased and that fit can be improved at the largely-curved lower section of the buttocks. Here, the arrangement interval denotes a distance between center positions in direction X of the respective elastic members.

Further, from viewpoints of easiness of following to the largely contracted and curved circumference of the waist similarly to the buttocks and less strange feeling with excellent fit and soft stiffness of the sheet member including the elastic members, it is preferable that the arrangement interval in the article longitudinal direction of the elastic members at the first region B1' of the rear body portion is equal to the arrangement interval in the article longitudinal direction of the elastic members at the lower extension portion B5'.

Here, being equal of the arrangement intervals includes a case that an absolute value of the difference between the arrangement intervals of both the above is 2 mm or less in addition to a case of being completely equal.

Examples of a method to differentiate a contraction stress in direction Y per unit length in direction X of a region of each of the front panel 2A and the rear panel 2B from that of another region thereof include (i) a method to differentiate stretch rates of elastic members between a region having a large contraction stress and a region having a small contraction stress when being fixed to the sheet 22 and/or the sheet 23, (ii) a method to differentiate thicknesses of elastic members between a region having a large contraction stress and a region having a small contraction stress, (iii) a method to differentiate materials of elastic members between a region having a large contraction stress and a region having a small contraction stress, (iv) a method to differentiate arrangement intervals of elastic members between a region having a large contraction stress and a region having a small contraction stress, and (v) a method in which two or more of the above are combined.

In the diaper 101 of the second embodiment, when the rear extension portion 21b is sectionalized into the upper rear extension portion and the lower rear extension portion by bisecting the rear extension portion 21b in the article longitudinal direction, it is preferable that the contraction stresses in the article lateral direction per unit length in the article longitudinal direction respectively at the upper rear extension portion and a portion of the front panel 2A corresponding to the upper rear extension portion are larger than the contraction stress of the lower rear extension portion.

Structures common to the diapers 1, 101 of the first and second embodiments will be described.

Each of the diapers 1, 101 of the first and second embodiments includes a waist stretchable portion G1 and a torso stretchable portion G2 at each of the body portions 20a, 20b of the front panel 2A and the rear panel 2B. Further, an extension stretchable portion G3 is formed at each of the extension portions 21a, 21b of the front panel 2A and the rear panel 2B.

Further, at each of the stretchable portions G1 to G3 of the front panel 2A and the rear panel 2B, the outer layer sheet 22 and the inner layer sheet 23 are joined at a number of fusion portions (joint portions) 26 formed discretely, and the elastic members 24 are arranged so as not to pass through the fusion portions 26.

More specifically, joint portion rows having the joint portions 26 arranged discontinuously and serially in the longitudinal direction (direction X) of the diaper 1, 101 are formed at the stretchable portions G1 to G3 into a plurality of rows in the lateral direction (direction Y) of the diaper 1, 101. Here, the respective joint portions of the joint portion rows are aligned in direction X.

Further, in each of the stretchable portions G1 to G3 of the front panel 2A and the rear panel 2B, a plurality of the elastic members 24 is arranged to pass between joint portions 26 which are adjacent in direction X, respectively. Further, each elastic member 24 is fixed between the sheets 22, 23 at a side joint region 27 or an absorbent assembly side joint region 28 described below while not being fixed to any of the sheets 22, 23 at a portion other than the above.

As illustrated in FIG. 3 or FIG. 8, in the front panel 2A, the side joint region 27 where the outer layer sheet 22 and the inner layer sheet 23 are joined with adhesive is arranged respectively at both the side edge portions 2a, 2a of the front panel or the vicinity thereof. Further, in the front panel 2A, the absorbent assembly side joint region 28 where the outer layer sheet 22 and the inner layer sheet 23 are joined with adhesive is arranged respectively at the center sides in the diaper lateral direction from the side joint region 27.

As illustrated in FIG. 4 or FIG. 9, in the rear panel 2B, the side joint region 27 where the outer layer sheet 22 and the inner layer sheet 23 are joined with adhesive is arranged respectively at both the side edge portions 2b, 2b of the rear panel or the vicinity thereof. Further, in the rear panel 2B as well, the absorbent assembly side joint region 28 where the outer layer sheet 22 and the inner layer sheet 23 are joined with adhesive is arranged respectively at the center sides in the diaper lateral direction from the side joint region 27.

Further, in each of the front panel 2A and the rear panel 2B, the elastic members 24 arranged at the waist stretchable portion G1 are fixed between the sheets 22, 23 respectively at a pair of the side joint regions 27 while not being fixed to any of the sheets 22, 23 between the side joint regions 27.

Meanwhile, in each of the front panel 2A and the rear panel 2B, the elastic members 24 arranged at the torso stretchable portion G2 and the extension stretchable portion G3 are fixed between the sheets 22, 23 respectively at the side joint regions 27 and the absorbent assembly side joint regions 28 while not being fixed to any of the sheets 22, 23 between the side joint region 27 and the absorbent assembly side joint region 28.

As illustrated in FIGs. 3 and 4 or FIGs. 8 and 9, the absorbent assembly side joint region 28 is formed so that an end thereof at the outer side in the diaper lateral direction is located at the inner side (center side) in the diaper lateral direction from a position of the side edge 3c of the absorbent assembly 3. Here, instead of the above, it is also possible to form across the inner side and the outer side of the side edge 3c of the absorbent assembly 3 or to form at the outer side in the diaper lateral direction from the side edge 3c of the absorbent assembly 3.

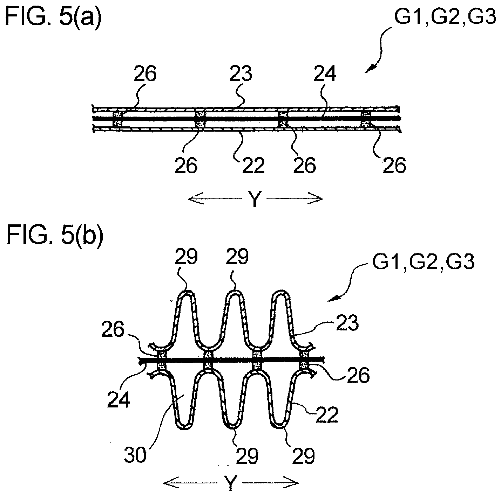

At the waist stretchable portion G1, the torso stretchable portion G2, and the extension stretchable portion G3 in each of the front panel 2A and the rear panel 2B, the sheets 22, 23 between adjacent joint portion rows are deformed to be swelled outward owing to contraction of the elastic members 24 and a gather 29 formed of the sheets 22, 23 is generated between adjacent joint portion rows. Further, a hollow portion 30 surrounded by the gathers 29, 29 is formed between both the sheets 22, 23 (see FIG. 5). Owing to that the gathers 29 and the hollow portions 30 are formed, texture and the like of the front panel 2A and the rear panel 2B are improved with improved softness. In addition, air is more likely to pass between the gathers 29 formed at a skin contacting face side and superior steam prevention characteristics can be obtained. In particular, in a case that the inner layer sheet 23 is formed of an air-permeable sheet such as nonwoven, humidity in the diaper 1, 101 is more easily ejected to the outside.

Meanwhile, as illustrated in FIGs. 3 and 4 or FIGs. 8 and 9, at each of the front panel 2A and the rear panel 2B in the diapers 1, 101 of the first and second embodiments, the side joint region 27 includes a portion having a larger width (length in direction Y) at a boundary region between the body portion 20a, 20b and the extension portion 21a, 21b than other portions.

More specifically, in the rear panel 2B of the diaper 1, a width W1 of the side joint region 27 at the third region B3 and the upper rear extension portion B4 is larger than a width W2 of the side joint region 27 at the first region B1, the second region B2, and the lower rear extension portion B5. In the front panel 2A of the diaper 1, the width W1 of the side joint region 27 at the third region A3 and the upper front extension portion A4 is larger than the width W2 of the side joint region 27 at the first region A1 and the second region A2.

Further, in the rear panel 2B of the diaper 101, a width W1 of the side joint region 27 at the third region B3' and the rear corresponding portion B4' is larger than a width W2 of the side joint region 27 at the first region B1', the second region B2', and the lower extension portion B5'. In the front panel 2A of the diaper 101, the width W1 of the side joint region 27 at the lower half portion of the third region A3' and the front extension portion A4' is larger than the width W2 of the side joint region 27 at the first region A1' and the second region A2'.

As described above, at the vicinity of the lower end of the side seal 4, the stress is prone to be increased especially with urination and a rubber imprint is prone to be left in relation with skin curvature and the like of a wearer.

In the diaper 1 of the first embodiment, owing to that the portion where the width of the side joint region 27 is large is arranged at the boundary region between the body portion 20a, 20b and the extension portion 21a, 21b, especially at the third region B3 and the upper rear extension portion B4 of the rear panel and/or the corresponding regions of the front panel, the portion is maintained to be flat compared to a portion where the gather 29 is formed and area contacting to skin is increased. Accordingly, it is possible to improve slip-drop prevention characteristics while preventing a rubber imprint from being left on skin while pressure due to the elastic members against skin is dispersed.

In the diaper 101 of the second embodiment, owing to that the portion where the width of the side joint region 27 is large is arranged at the boundary region between the body portion 20a, 20b and the extension portion A4', 21b, especially at the third region B3' and the rear corresponding portion B4' of the rear panel and/or the corresponding regions of the front panel, the portion is maintained to be flat compared to a portion where the gather 29 is formed and area contacting to skin is increased. Accordingly, it is possible to improve slip-drop prevention characteristics while preventing a rubber imprint from being left on skin while pressure due to the elastic members against skin is dispersed.

The total of the right and left widths W2 when the diaper is extended is in a range of 10% to 65% of a width of the front (or rear) sheet member in a stretched state preferably, and in a range of 15% to 60% more preferably. Further, for obtaining the abovementioned effects, the width W2 is in a range of 1.2 to 10 times of the width W1 preferably, and in a range of 4 to 7.5 times thereof more preferably.

The length L5 (see FIGs. 2 and 7) of the front extension portion 21a is in a range of 5% to 60% of the length La (see FIGs. 2 and 7) of the front panel 2A preferably, and in a range of 20% to 40% thereof more preferably. The length L6 (see FIGs. 2 and 7) of the rear extension portion 21b is in a range of 5% to 60% of the length Lb (see FIGs. 2 and 7) of the rear panel 2B preferably, and in a range of 20% to 40% thereof more preferably. Further, in a case of a diaper for an infant, the lengths L5, L6 of the front and rear extension portions 21a, 21b are in a range of 10 to 150 mm preferably, and in a range of 20 to 100 mm more preferably. In a case of a diaper for an adult, the lengths L5, L6 of the front and rear extension portions 21a, 21b are in a range of 10 to 200 mm preferably, and in a range of 20 to 150 more preferably.

From a viewpoint not to provide an impression that clearance between the front extension portion 21a and the rear extension portion 21b is caused by peeling at the side seal or splitting of the sheet, it is preferable that the length L6 of the rear extension portion 21b is longer than the length L5 of the front extension portion 21a (L6 > L5). Here, from viewpoints of fitting to a body shape and appearance, the length L6 of the rear extension portion 21b is in a range of 1.2 to 10 times of the length L5 of the front extension portion 21a preferably, and in a range of 1.5 to 6.5 times thereof more preferably. For example it is preferable from viewpoints of improving worn appearance such as fitting to a constricted and curved body shape and preventing protrusion of the buttocks and improving a strange feeling while worn.

The lengths L5, L6 of the front extension portion 21a and the rear extension portion 21b denote lengths in the diaper longitudinal direction (direction X).

Here, in the diaper 1, 101 of the first and second embodiments, the outer layer sheet 22 and the inner layer sheet 23 include extension folding portions 22a, 23a which are folded back onto the side of the inner layer sheet 23 at the edge portion 2c which forms the opening circumferential end of the waist opening. The extension folding portions 22a, 23a are joined, at the side seal 4, to portions of the outer layer sheet 22 and the inner layer sheet 23 which are not folded back. Further, portions thereof overlapped with the absorbent assembly 3 in the longitudinal direction are joined to a face of the absorbent assembly 3 on the side of the topsheet 31 with adhesive.

Although a variety of sheet materials which are conventionally used for such articles may be used as the outer layer sheet 22 and the inner layer sheet 23 of the diaper 1, 101 without specific limitation, nonwoven is preferable. Particularly, from viewpoints of flexibility and the like, it is preferable to adopt nonwoven with a single layer or layered nonwoven with two or more layers made of air-through nonwoven, heat-roll nonwoven, spanlaced nonwoven, spunbond nonwoven, meltblown nonwoven, or the like. Further, it is also possible to adopt a sheet integrating the nonwoven with a film. A variety of known elastic materials which are used for absorbent articles such as disposable diapers and sanitary pads may be used as materials for forming the elastic members 24 without specific limitation. Examples of elastic materials include synthetic rubber such as styrene-butadiene, butadiene, isoprene, and neoprene, natural rubber, EVA, stretchable polyolefin, and polyurethane. The elastic members used may be preferably thread-shaped (thread rubber or the like) with a section being rectangular, square, circular, polygonal or the like, string-shaped (flat rubber or the like), thread-shaped to be a multifilament type, or the like.

For example, a continuous-length original material for the front panel 2A and a continuous-length original material for the rear panel 2B are conveyed as being spaced and the absorbent bodies 3 are intermittently fixed as bridging between both thereof. Subsequently, heat sealing or the like (joining) for forming the side seals 4 is performed after two-folding causing the continuous-length original material for the front panel 2A and the continuous-length original material for the rear panel 2B to be overlapped. At the same time with or after the joining, cutting is performed to divide the above into an individual disposable diaper. In this manner, the abovementioned diaper 1, 101 can be manufactured effectively. Compared to a case to form penetrating holes or cutouts for forming leg openings, according to this method, trimming of an original material of an external material can be eliminated or portions to be trimmed can be lessened. Particularly, cutting the original materials for the front panel 2A and the rear panel 2B on a line perpendicular to a conveyance direction to form the front panel 2A and the rear panel 2B of the completed diaper 1, 101 is preferable because the trimming can be unnecessary.

In the above, the present invention is described based on the preferable embodiments. Here, not limited to the abovementioned embodiments, the present invention may be appropriately modified.

For example, instead of the dot-shaped joint portions 26 formed not to be overlapped with the elastic members 24, each of the front panel 2A and the rear panel 2B may have a structure in which the outer layer sheet 22 forming an external face of a diaper and the inner layer sheet 23 arranged at the inner side thereof are joined at the entire range. Further, in each of the waist stretchable portion G1, the torso stretchable portion G2, and the extension stretchable portion G3, the whole or a part thereof may have a structure in which the outer layer sheet 22 forming an external face of a diaper and the inner layer sheet 23 arranged at the inner side thereof are joined at the entire range.

Further, the torso stretchable portion G2 may be formed over the entire width of the front panel 2A and/or the rear panel 2B. The extension stretchable portion G3 may be formed over the entire width of the front panel 2A and/or the rear panel 2B as well.

Further, in the diaper 1 of the first embodiment, the length L5 in direction X of the front extension portion 21a may be larger than the length L61 in direction X of the upper rear extension portion B4. In this case, an elastic member may be or may not be arranged at a portion (a portion corresponding to the lower rear extension portion B5) extended below a portion (the upper front extension portion A4) corresponding to the upper rear extension portion B4. Further, in a case that the portion has stretchability in direction Y, a contraction stress thereof may be larger than, smaller than, or equal to that of the upper front extension portion A4. However, it is preferable to be equal to or smaller than that.

Further, although the side seal 4 is formed by joining the side edge portions 2a, 2b of the front and rear panels, it is also possible that a non-joint portion having a narrow width (e.g., more than 0 mm and equal to or less than 20 mm) where the front panel 2A and the rear panel 2B are not joined is formed at the outside of the side seal 4.

Further, other than a disposable pull-on diaper for an infant or an adult, the pull-on absorbent article may be a pull-on sanitary napkin, or the like.

Portions which are not described in any one of the abovementioned embodiments and elements included in only one of the abovementioned embodiments may be appropriately applied respectively to other embodiments. Further, elements in the respective embodiments are appropriately replaceable among the embodiments from one another.

In relation to the abovementioned embodiments, the present invention further discloses the following absorbent articles.

[1] A pull-on absorbent article having a waist opening and a pair of leg openings, including a rectangular front panel adapted to be worn about the front of a wearer, a rectangular rear panel adapted to be worn about the back of a wearer, and an absorbent assembly fixed as bridging between the front panel and the rear panel, wherein each of the front panel and the rear panel includes a body portion which includes side seals at both lateral sides and an extension portion which is extended from the body portion toward a crotch portion without including the side seals at both lateral sides, each of the body portion and the extension portion of the front panel and the body portion, an upper rear extension portion, and a lower rear extension portion of the rear panel has stretchability in the article lateral direction while the extension portion of the rear panel is sectionalized into the upper rear extension portion and the lower rear extension portion by bisecting the extension portion of the rear panel in the article longitudinal direction, and each of the upper rear extension portion and a portion of the front panel corresponding to the upper rear extension portion has a larger contraction stress in the article lateral direction per unit length in the article longitudinal direction than a contraction stress of the lower rear extension portion.

[2] The pull-on absorbent article according to subject [1], wherein a lower half portion of a third region of each of the front panel and the rear panel has a contraction stress in the article lateral direction per unit length in the article longitudinal direction being larger than a contraction stress of a first region and being smaller than a contraction stress of a second region, while the body portion is sectionalized into three regions by trisecting the side seal in the article longitudinal direction to be the first region, the second region, and the third region sequentially from a side close to the waist opening.

[3] The pull-on absorbent article according to subject 1 or subject 2, wherein the upper rear extension portion and/or a portion of the front panel corresponding to the upper rear extension portion have a contraction stress in the article lateral direction per unit length in the article longitudinal direction being smaller than a contraction stress of the second region of the rear panel and/or the front panel, while the body portion is sectionalized into three regions by trisecting the side seal in the longitudinal direction to be the first region, the second region, and the third region sequentially from a side close to the waist opening.

[4] The pull-on absorbent article according to any one of subjects [1] to [3], wherein the upper rear extension portion and/or a portion of the front panel corresponding to the upper rear extension portion have a contraction stress in the article lateral direction per unit length in the article longitudinal direction being equal to a contraction stress of a lower half portion of the third region of the rear panel and/or the front panel, while the body portion is sectionalized into three regions by trisecting the side seal in the longitudinal direction to be the first region, the second region, and the third region sequentially from a side close to the waist opening.

[5] The pull-on absorbent article according to subject [4], wherein the contraction stress of the lower half portion B32 of the third region B3 of the rear panel 2B is in a range of 0.8 to 1.2 times of the contraction stress of the upper rear extension portion B4.

[6] The pull-on absorbent article according to subject [4] or subject [5], wherein the contraction stress of the lower half portion A32 of the third region A3 of the front panel 2A is in a range of 0.8 to 1.2 times of the contraction stress of the upper front extension portion A4.

[7] The pull-on absorbent article according to any one of subjects [1] to [6], wherein the extension portion of the rear panel is longer than the extension portion of the front panel.

[8] The pull-on absorbent article according to any one of subjects [1] to [7], wherein a stretchable portion in which two sheets are mutually joined at a number of discretely-formed joint portions and in which each elastic member is arranged to pass between joint portions being adjacent in the article longitudinal direction is arranged at the body portion and the extension portion of each of the front panel and the rear panel.

[9] The pull-on absorbent article according to any one of subjects [1] to [8], wherein a joint region in which the two sheets are joined with adhesive is arranged at both side edges of each of the front panel and the rear panel or the vicinity thereof, and the joint region includes a portion having a larger width at a boundary region between the body portion and the extension portion than other portions.

[10] The pull-on absorbent article according to any one of subjects [1] to [9], wherein the waist stretchable portion G1 is formed at the outer side from the end part 3a, 3b in the longitudinal direction of the absorbent assembly 3 in the longitudinal direction (direction X) of the diaper 1 at each of the front panel 2A and the rear panel 2B.

[11] The pull-on absorbent article according to subject [10], wherein the torso stretchable portion G2 is formed between the waist stretchable portion G1 and the front extension portion 21a or the rear extension portion 21b in the longitudinal direction (direction) X of the diaper 1 at each of the front panel 2A and the rear panel 2B.

[12] The pull-on absorbent article according to any one of subjects [1] to [11], wherein the torso stretchable portion G2 is formed at least at positions being at the outer side respectively from both side edges in the longitudinal direction of the absorbent assembly 3 in the lateral direction (direction Y) of the pull-on absorbent article.

[13] The pull-on absorbent article according to any one of subjects [1] to [12], wherein the extension stretchable portion is formed at the extension portion of the front panel and/or the rear panel, and the extension stretchable portion is formed at least at positions being at the outer side from both side edges in the longitudinal direction of the absorbent assembly 3 in the lateral direction (direction Y) of the pull-on absorbent article.

[14] The pull-on absorbent article according to subject [10], wherein the waist stretchable portion G1 is configured to provide stretchability over the entire range between side joint regions 27, 27 of the front panel 2A or the rear panel 2B.

[15] The pull-on absorbent article according to any one of subjects [10] to [14], wherein the torso stretchable portion G2 and the extension stretchable portion G3 are configured to be in a state of being separated to the right and left of the pull-on absorbent article (diaper 1) so that stretchability is provided to the outer side from both the side edges of the absorbent assembly 3 and stretchability is not provided to a portion to be overlapped with the absorbent assembly 3, especially, to be overlapped with a center part in the lateral direction of the absorbent assembly 3.

[16] The pull-on absorbent article according to any one of subjects [1] to [15], wherein a plurality of the elastic members 24 is arranged at each of the front body portion 20a, the rear body portion 20b, the front extension portion 21a, and the rear extension portion 21b, respectively, in a stretched state at intervals in the diaper longitudinal direction as being extended along the diaper lateral direction.

[17] The pull-on absorbent article according to any one of subjects [1] to [16], wherein a length L5 in direction X of the front extension portion 21a is the same as a length L61 in direction X of the upper rear extension portion B4.

[18] The pull-on absorbent article according to any one of subjects [1] to [16], wherein a length L5 in direction X of the front extension portion 21a is larger than a length L61 in direction X of the upper rear extension portion B4.

[19] The pull-on absorbent article according to any one of subjects [1] to [16], wherein a length L5 in direction X of the front extension portion 21a is smaller than a length L61 in direction X of the upper rear extension portion B4.

[20] The pull-on absorbent article according to any one of subjects [1] to [19], wherein the contraction stress of the upper rear extension portion B4 is in a range of 1.3 to 3.5 times of the contraction stress of the lower rear extension portion B5, and in particular, in a range of 1.5 to 3 times thereof.

[21] The pull-on absorbent article according to any one of subjects [1] to [20], wherein the contraction stress of the upper rear extension portion B4 is in a rage of 14 to 35 cN, and in particular, in a range of 17 to 30 cN.

[22] The pull-on absorbent article according to any one of subjects [1] to [21], wherein the contraction stress of the lower rear extension portion B5 is in a range of 1 to 30 cN, and in particular, in a range of 5 to 25 cN.

[23] The pull-on absorbent article according to any one of subjects [1] to [22], wherein the contraction stress of the upper front extension portion A4 is in a range of 1.3 to 3.5 times of the contraction stress of the lower rear extension portion B5, and in particular, in a range of 1.5 to 3 times thereof.

[24] The pull-on absorbent article according to any one of subjects [1] to [23], wherein the contraction stress of the upper front extension portion A4 is in a range of 14 to 35 cN, and in particular, in a range of 17 to 30 cN.

[25] The pull-on absorbent article according to any one of subjects [1] to [24], wherein the contraction stress of the second region A2 is the largest among the first to third regions A1 to A3 of the front panel 2A.

[26] The pull-on absorbent article according to any one of subjects [1] to [25], wherein second regions A2, B2 are contacted to a portion from an iliac crest to an upper front iliac spine of a wearer while the pull-on absorbent article (diaper 1) is worn, while the body portion is sectionalized into three regions by trisecting the side seal in the longitudinal direction to be the first region, the second region, and the third region sequentially from a side close to the waist opening.

[27] The pull-on absorbent article according to any one of subjects [1] to [26], wherein the contraction stress of a second region B2, A2 in each of the rear panel 2B and the front panel 2A is in a range of 1.3 to 2.5 times of the contraction stress of a third region B3, A3, and in particular, in a range of 1.3 to 2 times thereof, while the body portion is sectionalized into three regions by trisecting the side seal in the longitudinal direction to be the first region, the second region, and the third region sequentially from a side close to the waist opening.

[28] The pull-on absorbent article according to any one of subjects [1] to [27], wherein the contraction stress of a second region B2, A2 in each of the rear panel 2B and the front panel 2A is in a range of 1.3 to 2.5 times of the contraction stress of a lower half portion B32, A32 of a third region, and in particular, in a range of 1.3 to 2 times thereof, while the body portion is sectionalized into three regions by trisecting the side seal in the longitudinal direction to be the first region, the second region, and the third region sequentially from a side close to the waist opening.

[29] The pull-on absorbent article according to any one of subjects [1] to [28], wherein the contraction stress of a lower half portion B32, A32 of a third region in each of the rear panel 2B and the front panel 2A is in a range of 1.2 to 3.5 times of the contraction stress of a first region B1, A1, and in particular, in a range of 1.2 to 3.0 times thereof, while the body portion is sectionalized into three regions by trisecting the side seal in the longitudinal direction to be the first region, the second region, and the third region sequentially from a side close to the waist opening.

[30] The pull-on absorbent article according to any one of subjects [1] to [29], wherein each of the front panel and the rear panel includes a side joint region where the an elastic member is fixed with adhesive between sheets at both side edges or the vicinity thereof, and the side joint region 27 includes a portion having a larger width (length in direction Y) at a boundary region between the body portion 20a, 20b and the extension portion 21a, 21b in each of the front panel 2A and the rear panel 2B than other portions.

[31] The pull-on absorbent article according to any one of subjects [1] to [30], wherein the rear panel includes a side joint region where the an elastic member is fixed with adhesive between sheets at both side edges or the vicinity thereof, and a width W1 of the side joint region 27 at the third region B3 and the upper rear extension portion B4 is larger than a width W2 of the side joint region 27 at the first region B1, the second region B2, and the lower rear extension portion B5.

[32] The pull-on absorbent article according to any one of subjects [1] to [31], wherein the front panel includes a side joint region where the an elastic member is fixed with adhesive between sheets at both side edges or the vicinity thereof, and a width W1 of the side joint region 27 at the third region A3 and the upper front extension portion A4 is larger than a width W2 of the side joint region 27 at the first region A1 and the second region A2.

[33] The pull-on absorbent article according to any one of subjects [1] to [32], wherein the front panel and the rear panel include front and rear body portions including side seals at both lateral sides and front and rear extension portions extended respectively from the front and rear body portions toward the crotch portion without having the side seals at both lateral sides, the rear extension portion has a length in the article longitudinal direction being the same as or larger than that of the front extension portion, each of the front body portion, the front extension portion, the rear body portion, and a rear corresponding portion of the rear extension portion corresponding to the front extension portion has stretchability in the article lateral direction owing to each elastic member arranged in the article lateral direction, and following relational expressions (11) to (14) are satisfied under definitions that $P_{A1'} \sim P_{A4'}$ denote contraction stresses in the article lateral direction per unit length in the article longitudinal direction and $Q_{A1'} \sim Q_{A4'}$ denote contraction stresses in the article lateral direction per single elastic member respectively of first to third regions A1' to A3' and the front extension portion A4' of the front panel, while the front body portion is sectionalized into three regions by trisecting the side seal in the article longitudinal direction to be the first region A1', the second region A2', and the third region A3' sequentially from a side close to the waist opening and definitions that $P_{B1'} \sim P_{B4'}$ denote contraction stresses in the article lateral direction per unit length in the article longitudinal direction and $Q_{B1'} \sim Q_{B4'}$ denote contraction stresses in the article lateral direction per single elastic member respectively of first to third regions B1' to B3' and the rear corresponding portion B4' of the rear panel, while the rear body portion is sectionalized into three regions by trisecting the side seal in the article longitudinal direction to be the first region B1', the second region B2', and the third region B3' sequentially from a side close to the waist opening.

$$\text{Ratio } (P_{A3'}/P_{A1'}) > \text{Ratio } (Q_{A3'}/Q_{A1'}), \text{Ratio } (P_{A3'}/P_{A1'}) > 1 \ldots (11)$$

$$\text{Ratio } (P_{B3'}/P_{B1'}) > \text{Ratio } (Q_{B3'}/Q_{B1'}), \text{Ratio } (P_{B3'}/P_{B1'}) > 1 \ldots (12)$$

$$\text{Ratio } (P_{A4'}/P_{A1'}) > \text{Ratio } (Q_{A4'}/Q_{A1'}), \text{Ratio } (P_{A4'}/P_{A1'}) > 1 \ldots (13)$$

$$\text{Ratio } (P_{B4'}/P_{B1'}) > \text{Ratio } (Q_{B4'}/Q_{B1'}), \text{Ratio } (P_{B4'}/P_{B1'}) > 1 \ldots (14)$$

[34] The pull-on absorbent article according to any one of subjects [1] to [33], wherein each of the ratio $(P_{A3'}/A1')$, the ratio $(P_{B3'}/P_{B1'})$, the ratio $(P_{A4'}/P_{A1'})$, and the ratio $(P_{B4'}/P_{B1'})$ is in a range of 1.5 to 7.0 preferably, in a range of 1.6 to 5.0 more preferably, and in a range of 1.7 to 3.5 even more preferably.

[35] The pull-on absorbent article according to any one of subjects [1] to [34], wherein each of the ratio $(Q_{A3'}/Q_{A1'})$, the ratio $(Q_{B3'}/Q_{B1'})$, the ratio $(Q_{A4'}/Q_{A1'})$, and the ratio $(Q_{B4'}/Q_{B1'})$ is in a range of 0.3 to 2.2 preferably, in a range of 0.5 to 1.8 more preferably, and in a range of 0.7 to 1.4 even more preferably.

[36] The pull-on absorbent article according to any one of subjects [33] to [35], wherein following relational expressions (15) and (16) are satisfied.

$$P_{A3'} = P_{A4'}, Q_{A3'} = Q_{A4'} \quad \ldots \quad (15)$$

$$P_{B3'} = P_{B4'}, Q_{B3'} = Q_{B4'} \quad \ldots \quad (16)$$

[37] The pull-on absorbent article according to any one of subjects [33] to [36], wherein following relational expressions (17) and (18) are satisfied.

$$4 \text{ cN} < Q_{A3'} < 23 \text{ cN} \quad \ldots \quad (17)$$

$$4 \text{ cN} < Q_{B3'} < 23 \text{ cN} \quad \ldots \quad (18)$$

[38] The pull-on absorbent article according to any one of subjects [33] to [37], wherein following relational expressions (19) and (20) are satisfied.

$$P_{A2'} > P_{A3'}, P_{B2'} > P_{B3'} \quad \ldots \quad (19)$$

$$P_{A2'} > P_{A4'}, P_{B2'} > P_{B4'} \quad \ldots \quad (20)$$

[39] The pull-on absorbent article according to any one of subjects [33] to [38], wherein the rear extension portion includes a lower extension portion B5' which is extended further toward the crotch portion than the rear corresponding portion, the lower extension portion has stretchability in the article lateral direction owing to an elastic member arranged in the article lateral direction, and a following relational expression (21) is satisfied under definitions that $P_{B5'}$ denotes a contraction stress in the article lateral direction per unit length in the article longitudinal direction and $Q_{B5'}$ denotes a contraction stress in the article lateral direction per single elastic member respectively of the lower extension portion.

$$\text{Ratio } (P_{B4'}/P_{B5'}) > \text{Ratio } (Q_{B4'}/Q_{B5'}), \text{Ratio } (P_{B4'}/P_{B5'}) > 1 \ldots (21)$$

[40] The pull-on absorbent article according to any one of subjects [33] to [39], wherein an arrangement interval in the article longitudinal direction of the elastic members in the second and third regions A2', A3' and the front extension portion A4' of the front panel is smaller than an arrangement interval in the article longitudinal direction of the elastic members in the first region A1' of the front panel.

[41] The pull-on absorbent article according to any one of subjects [33] to [40], wherein an arrangement interval in the article longitudinal direction of the elastic members in the second and third regions B2', B3' and the rear corresponding portion B4' of the rear panel is smaller than an arrangement interval in the article longitudinal direction of the elastic members in the first region B1' of the rear panel.

[42] The pull-on absorbent article according to any one of subjects [33] to [41], wherein the rear extension portion includes a lower extension portion B5' which is extended further toward the crotch portion than the rear corresponding portion, each of the rear corresponding portion and the lower extension portion has stretchability in the article lateral direction owing to a plurality of elastic members arranged in the article lateral direction, and an arrangement interval in the article longitudinal direction of the elastic members in the rear corresponding portion is larger than an arrangement interval in the article longitudinal direction of the elastic members in the lower extension portion.

[43] The pull-on absorbent article according to any one of subjects [33] to [42], wherein the rear extension portion includes a lower extension portion B5' which is extended further toward the crotch portion than the rear corresponding portion, each of the first region of the rear body portion and the lower extension portion has stretchability in the article lateral direction owing to a plurality of elastic members arranged in the article lateral direction, and an arrangement interval in the article longitudinal direction of the elastic members in the lower extension portion is equal to an arrangement interval in the article longitudinal direction of the elastic members in the first region of the rear body portion.

[44] The pull-on absorbent article according to any one of subjects [33] to [43], wherein a stretchable portion in which two sheets are mutually joined at a number of discretely-formed joint portions and in which each elastic member is arranged to pass between joint portions being adjacent in the article longitudinal direction is arranged at the body portion and the extension portion of each of the front panel and the rear panel.

[45] The pull-on absorbent article according to any one of subjects [1] to [44], wherein the pull-on absorbent article is a disposable pull-on diaper for an infant.

Examples

In the following, the present invention will be described more specifically with examples. Here, the present invention is not limited by the following disclosure.

[Example 1 and comparison examples 1 and 2]

Underwear-type disposable diapers shaped as illstrated in FIGs. 1 and 2 were prepared as each portion having a contraction stress indicated in table 1 and elastic members in table 1 being arranged at a front panel and a rear panel under conditions in table 1. A Spandex continuous-length elastic body was adopted as each of the elastic members.

In tables 1 and 2, a mark of "↑" indicates being the same as the above. Further, the length L5 in direction X of the front extension portion 21a in the diaper of example 1 was set to a half of the length L6 in direction X of the rear extension portion 21b (being the same as the length L61 in direction X of the upper rear extension portion B4). Comparison examples 1 and 2 were prepared similarly to the above. A contraction stress of each portion denotes a contraction stress per unit length (10 mm) in the diaper longitudinal direction.

[Table 1-1] (continued)

| | | Region segment | | Length of each region in direction X | Elastic member | | | Contraction stress |
| | | | | | Thickness | Arrangement interval | Stretch rate | |
| | | | | mm | dtex | mm | Magnification | cN/10 mm |
|---|---|---|---|---|---|---|---|---|
| Example 1 | Front sheet member | First region A1 | | 40 | 310 | 6.5 | 3.0 | 16.9 |
| | | Second region A2 | | ↑ | 940 | ↑ | ↑ | 35.3 |
| | | Third region A3 | Upper half portion A31 | 20 | 310 | ↑ | ↑ | 13.9 |
| | | | Lower half portion A32 | ↑ | 620 | ↑ | ↑ | 26.1 |
| | | | Total | 40 | 310/620 | ↑ | ↑ | 20.0 |
| | | Upper front extension portion A4 | | 25 | 620 | ↑ | ↑ | 26.1 |
| | Rear sheet member | First region A1 | | 40 | 310 | ↑ | ↑ | 16.9 |
| | | Second reg on A2 | | ↑ | 940 | ↑ | ↑ | 35.3 |
| | | Third region A3 | Upper half portion A31 | 20 | 310 | ↑ | ↑ | 13.9 |
| | | | Lower half portion A32 | ↑ | 620 | ↑ | ↑ | 26.1 |
| | | | Total | 40 | 310/620 | ↑ | ↑ | 20.0 |
| | | Upper rear extension portion B4 | | 25 | 620 | ↑ | ↑ | 26.1 |
| | | Lower rear extension portion B5 | | ↑ | 310 | ↑ | ↑ | 13.9 |

EP 2 659 870 B1

(continued)

| | | Region segment | | Length of each region in direction X | Elastic member | | | Contraction stress |
|---|---|---|---|---|---|---|---|---|
| | | | | | Thickness | Arrangement interval | Stretch rate | |
| | | | | mm | dtex | mm | Magnification | cN/10 mm |
| Example 2 | Front sheet member | First region A1 | | 40 | 310 | ↑ | ↑ | 16.9 |
| | | Second region A2 | | ↑ | 620 | ↑ | ↑ | 26.1 |
| | | Third region A3 | Upper half portion A31 | 20 | 620 | ↑ | ↑ | 26.1 |
| | | | Lower half portion A32 | ↑ | 620 | ↑ | ↑ | 26.1 |
| | | | Total | 40 | 620 | ↑ | ↑ | 26.1 |
| | | Upper front extension portion A4 | | 25 | 620 | ↑ | ↑ | 26.1 |
| | Rear sheet member | First region A1 | | 40 | 310 | ↑ | ↑ | 16.9 |
| | | Second region A2 | | ↑ | 620 | ↑ | ↑ | 26.1 |
| | | Third region A3 | Upper half portion A31 | 20 | 620 | ↑ | ↑ | 26.1 |
| | | | Lower half portion A32 | ↑ | 620 | ↑ | ↑ | 26.1 |
| | | | Total | 40 | 620 | ↑ | ↑ | 26.1 |
| | | Upper rear extension portion B4 | | 25 | 620 | ↑ | ↑ | 26.1 |
| | | Lower rear extension portion B5 | | ↑ | 310 | ↑ | ↑ | 13.9 |

**EP 2 659 870 B1**

[Table 1-2]

| | | | | | | | | |
|---|---|---|---|---|---|---|---|---|
| Comparative example 1 | Front sheet member | First region A1 | | 40 | 310 | ↑ | ↑ | 16.9 |
| | | Second region A2 | | ↑ | 940 | ↑ | ↑ | 35.3 |
| | | Third region A3 | Upper half portion A31 | 20 | 310 | ↑ | ↑ | 13.9 |
| | | | Lower half portion A32 | ↑ | 940 | ↑ | ↑ | 35.3 |
| | | | Total | 40 | 310/940 | ↑ | ↑ | 24.6 |
| | | Upper front extension portion A4 | | 25 | 310 | ↑ | ↑ | 13.9 |
| | Rear sheet member | First region A1 | | 40 | 310 | ↑ | ↑ | 16.9 |
| | | Second region A2 | | ↑ | 940 | ↑ | ↑ | 35.3 |
| | | Third region A3 | Upper half portion A31 | 20 | 310 | ↑ | ↑ | 13.9 |
| | | | Lower half portion A32 | ↑ | 940 | ↑ | ↑ | 35.3 |
| | | | Total | 40 | 310/940 | ↑ | ↑ | 24.6 |
| | | Upper rear extension portion B4 | | 25 | 310 | ↑ | ↑ | 13.9 |
| | | Lower rear extension portion B5 | | ↑ | 310 | ↑ | ↑ | 13.9 |
| Comparative example 2 | Front sheet member | First region A1 | | 40 | 310 | ↑ | ↑ | 16.9 |
| | | Second region A2 | | ↑ | 940 | ↑ | ↑ | 35.3 |
| | | Third region A3 | Upper half portion A31 | 20 | 310 | t | ↑ | 13.9 |
| | | | Lower half portion A32 | ↑ | 310 | ↑ | ↑ | 13.9 |
| | | | Total | 40 | 310 | ↑ | ↑ | 13.9 |
| | | Upper front extension portion A4 | | 25 | 310 | ↑ | ↑ | 13.9 |
| | Rear sheet member | First region A1 | | 40 | 310 | ↑ | ↑ | 16.9 |
| | | Second | region A2 | ↑ | 940 | ↑ | ↑ | 35.3 |
| | | Third region A3 | Upper half portion A31 | 20 | 310 | ↑ | ↑ | 13.9 |
| | | | Lower half portion A32 | ↑ | 310 | ↑ | ↑ | 13.9 |
| | | | Total | 40 | 310 | ↑ | ↑ | 13.9 |
| | | Upper rear extension portion B4 | | 25 | 310 | ↑ | ↑ | 13.9 |
| | | Lower rear extension portion B5 | | ↑ | 310 | ↑ | ↑ | 13.9 |

[Evaluation]

The prepared disposable pull-on diapers were evaluated with the following method on (1) resistance to slip-drop and (2) resistance to rubber imprint about thighs. Table 2 indicates the result of the above.

(1) Resistance to slip-drop

Simulated urine of 240 g (assumption of awake conditions) was poured into a diaper of each of examples 1 and 2 and comparison examples 1 and 2 worn to a dynamic baby model corresponding to one to two years old. Comparison was performed on slip-drop amounts about thighs after motion of 30 minutes. A length from an intersection point between the groin and a tragus vertical line (groin side point) to a side seal lower end of a diaper was measured as the slip-drop amount about thighs.

(Evaluation reference)

○: Slip-drop amount at thighs being less than 15 mm.

Δ: Slip-drop amount at thighs being 15 mm or more and less than 25 mm.

×: Slip-drop amount at thighs being 25 mm or more.

(2) Resistance to rubber imprint about thighs

A diaper of each of examples 1 and 2 and comparison examples 1 and 2 was worn to an infant being one to two years old for four to ten hours (in normal usage) and appearance of rubber imprints was observed.

(Evaluation reference)

○: Level with rubber imprints being hardly seen.

Δ: Level with rubber imprints being barely-troublesome.

×: Level with sharp rubber imprints being troublesome.

[Table 2]

|  | Resistance to slip-drop | Rubber imprint about thighs |
| --- | --- | --- |
| Example 1 | ○ | ○ |
| Example 2 | ○ | ○ |
| Comparative example 1 | Δ | × |
| Comparative example 2 | × | ⊙ |

From the results indicated in table 2, it is proved, owing to setting the contraction stress of the upper rear extension portion B4 and the contraction stress of the portion corresponding to the upper rear extension portion B4 of the front panel (upper front extension portion A4) to be larger than the contraction stress of the lower rear extension portion, that rubber imprints can be prevented from being left at the vicinity of the waist while effectively preventing diaper slippage without excessive increase of the stress of the third region where rubber imprints are prone to be left, in addition to that fit can be improved at the curved buttocks.

Further, from comparison between results of examples 1 and 2, it is proved, owing to setting the stress of the upper extension portion to be equal to the contraction stress of the lower half portion of the third region, that diaper slippage can be effectively prevented and rubber imprints can be prevented as well without excessive increase of the contraction stress of the third region where rubber imprints are prone to be left.

[Example 11 and comparative example 11]

Underwear-type disposable diapers generally shaped as illustrated in FIGs. 6 to 9 were prepared as each portion having a contraction stress indicated in table 3 and elastic members in table 3 being arranged at a front panel and a rear panel under conditions in table 3. A Spandex continuous-length elastic body was adopted as each of the elastic members.

In table 3, a mark of "↑" indicates being the same as the above. Further, the length L5 in direction X of the front extension portion 21a (A4') in the diaper of example 11 was set to a half of the length L6 in direction X of the rear extension portion 21b. Further, comparative example 11 was also set similarly to the above.

[Table 3]

| | | Region segment | Length of each region in direction X | Elastic member | | | Contraction stress per unit length | | Contraction stress per single elastic member | |
|---|---|---|---|---|---|---|---|---|---|---|
| | | | | Thickness | Arrangement interval | Stretch rate | | | | |
| | | | mm | dtex | mm | Magnification | (cN/10mm) | | (cN/strip) | |
| Example 11 | Front sheet member | First region A1' | 37 | 310 | 6.5 | 3 | $P_{A1'}$ | 19.1 | $Q_{A1'}$ | 11.5 |
| | | Second region A2' | 37 | 620 | 4 | ↑ | $P_{A2'}$ | 35.6 | $Q_{A2'}$ | 14.2 |
| | | Third region A3' | 37 | 470 | ↑ | ↑ | $P_{A3'}$ | 29.0 | $Q_{A3'}$ | 11.6 |
| | | Front extension portion A4' | 25 | ↑ | ↑ | ↑ | $P_{A4'}$ | 29.0 | $Q_{A4'}$ | 11.6 |
| | Rear sheet member | First region B1' | 37 | 310 | 6.5 | ↑ | $P_{B1'}$ | 19.1 | $Q_{B1'}$ | 11.5 |
| | | Second region B2' | 37 | 620 | 4 | ↑ | $P_{B2'}$ | 35.6 | $Q_{B2'}$ | 14.2 |
| | | Third region B3' | 37 | 470 | ↑ | ↑ | $P_{B3'}$ | 29.0 | $Q_{B3'}$ | 11.6 |
| | | Rear corresponding portion B4' | 25 | ↑ | ↑ | ↑ | $P_{B4'}$ | 29.0 | $Q_{B4'}$ | 11.6 |
| | | Lower extension portion B5' | 25 | 470 | 6.5 | ↑ | $P_{B5'}$ | 17.8 | $Q_{B5'}$ | 11.6 |

(continued)

| | | Region segment | Length of each region in direction X | Elastic member | | | Contraction stress per unit length | | Contraction stress per single elastic member | |
|---|---|---|---|---|---|---|---|---|---|---|
| | | | | Thickness | Arrangement interval | Stretch rate | | | | |
| | | | mm | dtex | mm | Magnification | (cN/10mm) | | (cN/strip) | |
| Comparative example 11 | Front sheet member | First region A1' | 37 | 310 | 6.5 | ↑ | $P_{A1'}$ | 19.1 | $Q_{A1'}$ | 11.5 |
| | | Second region A2' | 37 | 940 | ↑ | ↑ | $P_{A2'}$ | 36.5 | $Q_{A2'}$ | 23.7 |
| | | Third region A3' | 37 | 620 | ↑ | ↑ | $P_{A3'}$ | 26.9 | $Q_{A3'}$ | 17.5 |
| | | Front extension portion A4' | 25 | ↑ | ↑ | ↑ | $P_{A4'}$ | 26.9 | $Q_{A4'}$ | 17.5 |
| | Rear sheet member | First region B1' | 37 | 310 | ↑ | ↑ | $P_{B1'}$ | 19.1 | $Q_{B1'}$ | 11.5 |
| | | Second region B2' | 37 | 940 | ↑ | ↑ | $P_{B2'}$ | 36.5 | $Q_{B2'}$ | 23.7 |
| | | Third region B3' | 37 | 620 | ↑ | ↑ | $P_{B3'}$ | 26.9 | $Q_{B3'}$ | 17.5 |
| | | Rear corresponding portion B4' | 25 | ↑ | ↑ | ↑ | $P_{B4'}$ | 26.9 | $Q_{B4'}$ | 17.5 |
| | | Lower extension portion B5' | 25 | ↑ | ↑ | ↑ | $P_{B5'}$ | 26.9 | $Q_{B5'}$ | 17.5 |

Arrangement interval: Length from the center of an elastic member to the center of an adjacent elastic member.
Stretch rate: Stretch magnification as an unstretched state being denoted by one.

Table 4 indicates ratios among the respective contraction stresses in example 11 and comparative example 11.

[Table 4]

| | | Ratio between contraction stresses | |
|---|---|---|---|
| Example 11 | Ratio in expression (11) | $(P_{A3'}/P_{A1'})$ | 1.52 |
| | | $(Q_{A3'}/Q_{A1'})$ | 1.01 |
| | Ratio in expression (12) | $(P_{B3'}/P_{B1'})$ | 1.52 |
| | | $(Q_{B3'}/Q_{B1'})$ | 1.01 |
| | Ratio in expression (13) | $(P_{A4'}/P_{A1'})$ | 1.52 |
| | | $(Q_{A4'}/Q_{A1'})$ | 1.01 |
| | Ratio in expression (14) | $(P_{B4'}/P_{B1'})$ | 1.52 |
| | | $(Q_{B4'}/Q_{B1'})$ | 1.01 |
| Comparative example 11 | Ratio in expression (11) | $(P_{A3'}/P_{A1'})$ | 1.41 |
| | | $(Q_{A3'}/Q_{A1'})$ | 1.52 |
| | Ratio in expression (12) | $(P_{B3'}/P_{B1'})$ | 1.41 |
| | | $(Q_{B3'}/Q_{B1'})$ | 1.52 |
| | Ratio in expression (13) | $(P_{A4'}/P_{A1'})$ | 1.41 |
| | | $(Q_{A4'}/Q_{A1'})$ | 1.52 |
| | Ratio in expression (14) | $(P_{B4'}/P_{B1'})$ | 1.41 |
| | | $(Q_{B4'}/Q_{B1'})$ | 1.52 |

[Evaluation]

The prepared disposable pull-on diapers were evaluated with the following method on (1) resistance to slip-drop and (2) resistance to rubber imprint about thighs. Table 5 indicates the result of the above.

[Table 5]

| | Resistance to slip-drop | Rubber imprint about thighs |
|---|---|---|
| Example 11 | ○ | ○ |
| Comparative example 11 | ○ | × |

From the results indicated in table 5, it is proved, according to the diaper (in particular, a pull-on absorbent article satisfying the abovementioned relational expressions (11) to (14)) of example 11 of the present invention, that pressing imprints of the elastic members are less likely to be left and a burden to skin is small with excellent skip-drop preventing characteristics while being a pull-on absorbent article having the external material divided into the front panel and the rear panel.

Industrial Applicability

A pull-on absorbent article of the present invention excellently covers the buttocks and causes less slip-drop and appearance worsening while worn as being a pull-on absorbent article capable of being effectively manufactured with an external material divided into a front panel and a rear panel.
Further, according to a pull-on absorbent article of the present invention, in particular, a pull-on absorbent article satisfying the abovementioned relational expressions (11) to (14), pressing imprints of elastic members are less likely to be left and a burden to skin is small.

**Claims**

1. A pull-on absorbent article having a waist opening and a pair of leg openings, comprising:

    a rectangular front panel adapted to be worn around a wearer's front;
    a rectangular rear panel adapted to be worn around a wearer's rear; and
    an absorbent assembly fixed to the front panel and the rear panel so as to bridge them,
    wherein each of the front panel and the rear panel includes

        a body portion which includes side seals located at both lateral sides of the panels; and
        an extension portion which is extended from the body portion toward a crotch portion and which has no side seals at both lateral sides of the panels,

        each of the body portion and the extension portion of the front panel and the body portion, an upper rear extension portion, and a lower rear extension portion of the rear panel has stretchability in the article lateral direction while the extension portion of the rear panel is sectionalized into the upper rear extension portion and the lower rear extension portion by bisecting the extension portion of the rear panel in the article longitudinal direction, and each of the upper rear extension portion and a portion of the front panel corresponding to the upper rear extension portion has a larger contraction stress in the article lateral direction per unit length in the article longitudinal direction than a contraction stress of the lower rear extension portion.

2. The pull-on absorbent article according to claim 1, wherein a lower half portion of a third region of each of the front panel and the rear panel has a contraction stress in the article width direction per unit length in the article longitudinal direction being larger than a contraction stress of a first region and being smaller than a contraction stress of a second region, while the body portion is sectionalized into three regions by trisecting the side seal in the article longitudinal direction to be the first region, the second region, and the third region sequentially from a side close to the waist opening.

3. The pull-on absorbent article according to claim 1 or claim 2, wherein the upper rear extension portion and/or a portion of the front panel corresponding to the upper rear extension portion have a contraction stress in the article lateral direction per unit length in the article longitudinal direction being smaller than a contraction stress of the second region of the rear panel and/or the front panel, while the body portion is sectionalized into three regions by trisecting the side seal in the longitudinal direction to be the first region, the second region, and the third region sequentially from a side close to the waist opening.

4. The pull-on absorbent article according to any one of claims 1 to 3, wherein the upper rear extension portion and/or a portion of the front panel corresponding to the upper rear extension portion have a contraction stress in the article lateral direction per unit length in the article longitudinal direction being equal to a contraction stress of a lower half portion of the third region of the rear panel and/or the front panel, while the body portion is sectionalized into three regions by trisecting the side seal in the longitudinal direction to be the first region, the second region, and the third region sequentially from a side close to the waist opening.

5. The pull-on absorbent article according to any one of claims 1 to 4, wherein the extension portion of the rear panel is longer than the extension portion of the front panel.

6. The pull-on absorbent article according to any one of claims 1 to 5, wherein a stretchable portion in which two sheets are mutually joined at a number of discretely-formed joint portions and in which each elastic member is arranged to pass between joint portions being adjacent in the article longitudinal direction is arranged at the body portion and the extension portion of each of the front panel and the rear panel.

7. The pull-on absorbent article according to any one of claims 1 to 6,
    wherein a joint region in which the two sheets are joined with adhesive is arranged at both side edges of each of the front panel and the rear panel or the vicinity thereof, and
    the joint region includes a portion having a larger width at a boundary region between the body portion and the extension portion than other portions.

8. The pull-on absorbent article according to any one of claims 1 to 7,
    wherein the front panel and the rear panel include front and rear body portions including side seals at both lateral sides and front and rear extension portions extended respectively from the front and rear body portions toward the crotch portion having no side seals at both lateral sides,

the rear extension portion has a length in the article longitudinal direction being the same as or larger than that of the front extension portion,

each of the front body portion, the front extension portion, the rear body portion, and a rear corresponding portion of the rear extension portion corresponding to the front extension portion has stretchability in the article lateral direction owing to each elastic member arranged in the article lateral direction, and

following relational expressions (11) to (14) are satisfied under definitions that $P_{A1'}{\sim}P_{A4'}$ denote contraction stresses in the article lateral direction per unit length in the article longitudinal direction and $Q_{A1'}{\sim}Q_{A4'}$ denote contraction stresses in the article lateral direction per single elastic member respectively of first to third regions A1' to A3' and the front extension portion A4' of the front panel, while the front body portion is sectionalized into three regions by trisecting the side seal in the article longitudinal direction to be the first region A1', the second region A2', and the third region A3' sequentially from a side close to the waist opening and definitions that $P_{B1'}{\sim}P_{B4'}$ denote contraction stresses in the article lateral direction per unit length in the article longitudinal direction and $Q_{B1'}{\sim}Q_{B4'}$ denote contraction stresses in the article lateral direction per single elastic member respectively of first to third regions B1' to B3' and the rear corresponding portion B4' of the rear panel, while the rear body portion is sectionalized into three regions by trisecting the side seal in the article longitudinal direction to be the first region B1', the second region B2', and the third region B3' sequentially from a side close to the waist opening.

$$\text{Ratio } (P_{A3'}/P_{A1'}) > \text{Ratio } (Q_{A3'}/Q_{A1'}), \text{ Ratio } (P_{A3'}/P_{A1'}) > 1 \ldots (11)$$

$$\text{Ratio } (P_{B3'}/P_{B1'}) > \text{Ratio } (Q_{B3'}/Q_{B1'}), \text{ Ratio } (P_{B3'}/P_{B1'}) > 1 \ldots (12)$$

$$\text{Ratio } (P_{A4'}/P_{A1'}) > \text{Ratio } (Q_{A4'}/Q_{A1'}), \text{ Ratio } (P_{A4'}/P_{A1'}) > 1 \ldots (13)$$

$$\text{Ratio } (P_{B4'}/P_{B1'}) > \text{Ratio } (Q_{B4'}/Q_{B1'}), \text{ Ratio } (P_{B4'}/P_{B1'}) > 1 \ldots (14)$$

9. The pull-on absorbent article according to claim 8, wherein following relational expressions (15) and (16) are satisfied.

$$P_{A3'} = P_{A4'}, \, Q_{A3'} = Q_{A4'} \quad \ldots \quad (15)$$

$$P_{B3'} = P_{B4'}, \, Q_{B3'} = Q_{B4'} \quad \ldots \quad (16)$$

10. The pull-on absorbent article according to claim 8 or claim 9, wherein following relational expressions (17) and (18) are satisfied.

$$4 \text{ cN} < Q_{A3'} < 23 \text{ cN} \quad \ldots \quad (17)$$

$$4 \text{ cN} < Q_{B3'} < 23 \text{ cN} \quad \ldots \quad (18)$$

11. The pull-on absorbent article according to any one of claims 8 to 10, wherein following relational expressions (19) and (20) are satisfied.

$$P_{A2'} > P_{A3'}, \, P_{B2'} > P_{B3'} \quad \ldots \quad (19)$$

$$P_{A2'} > P_{A4'}, \, P_{B2'} > P_{B4'} \quad \ldots \quad (20)$$

**12.** The pull-on absorbent article according to any one of claims 8 to 11,
wherein the rear extension portion includes a lower extension portion B5' which is extended from the rear corresponding portion toward the crotch portion, the lower extension portion has stretchability in the article lateral direction owing to an elastic member arranged in the article lateral direction, and
a following relational expression (21) is satisfied under definitions that $P_{B5'}$ denotes a contraction stress of the lower extension portion in the article lateral direction per unit length in the article longitudinal direction and $Q_{B5'}$ denotes a contraction stress in the article lateral direction per single elastic member respectively of the lower extension portion.

$$\text{Ratio } (P_{B4'}/P_{B5'}) > \text{Ratio } (Q_{B4'}/Q_{B5'}), \text{ Ratio } (P_{B4'}/P_{B5'}) > 1 \ldots (21)$$

**13.** The pull-on absorbent article according to any one of claims 8 to 12, wherein an arrangement interval in the article longitudinal direction of the elastic members in the second and third regions A2', A3' and the front extension portion A4' of the front panel is smaller than an arrangement interval in the article longitudinal direction of the elastic members in the first region A1' of the front panel.

**14.** The pull-on absorbent article according to any one of claims 8 to 13, wherein an arrangement interval in the article longitudinal direction of the elastic members in the second and third regions B2', B3' and the rear corresponding portion B4' of the rear panel is smaller than an arrangement interval in the article longitudinal direction of the elastic members in the first region B1' of the rear panel.

**15.** The pull-on absorbent article according to any one of claims 8 to 14,
wherein the rear extension portion includes a lower extension portion B5' which is extended from the rear corresponding portion toward the crotch portion,
each of the rear corresponding portion and the lower extension portion has stretchability in the article lateral direction owing to a plurality of elastic members arranged in the article lateral direction, and
an arrangement interval in the article longitudinal direction of the elastic members in the rear corresponding portion is larger than an arrangement interval in the article longitudinal direction of the elastic members in the lower extension portion.

**16.** The pull-on absorbent article according to any one of claims 8 to 15,
wherein the rear extension portion includes a lower extension portion B5' which is extended from the rear corresponding portion toward the crotch portion,
each of the first region of the rear body portion and the lower extension portion has stretchability in the article lateral direction owing to a plurality of elastic members arranged in the article lateral direction, and
an arrangement interval in the article longitudinal direction of the elastic members in the lower extension portion is equal to an arrangement interval in the article longitudinal direction of the elastic members in the first region of the rear body portion.

**17.** The pull-on absorbent article according to any one of claims 8 to 16, wherein a stretchable portion in which two sheets are mutually joined at a number of discretely-formed joint portions and in which each elastic member is arranged to pass between joint portions being adjacent in the article longitudinal direction is arranged at the body portion and the extension portion of each of the front panel and the rear panel.

**Patentansprüche**

**1.** Saugfähiger Schlupfartikel mit einer Taillenöffnung und einem Paar Beinöffnungen, der aufweist:

ein rechteckiges vorderes Feld, das geeignet ist, um um die Vorderseite eines Trägers getragen zu werden;
ein rechteckiges hinteres Feld, das geeignet ist, um um die Rückseite eines Trägers getragen zu werden; und
eine saugfähige Anordnung, die am vorderen Feld und am hinteren Feld befestigt ist, um sie zu überbrücken,
wobei das vordere Feld und das hintere Feld jeweils aufweisen:

einen Körperabschnitt, der Seitendichtungen aufweist, die an beiden Seitenflächen der Felder liegen; und
einen Verlängerungsabschnitt, der sich vom Körperabschnitt zu einem Schrittabschnitt erstreckt und der keine Seitendichtungen an beiden Seitenflächen der Felder hat,

der Körperabschnitt und der Verlängerungsabschnitt des vorderen Felds sowie der Körperabschnitt, ein oberer hinterer Verlängerungsabschnitt und ein unterer hinterer Verlängerungsabschnitt des hinteren Felds jeweils Dehnbarkeit in Artikelseitenrichtung haben, während der Verlängerungsabschnitt des hinteren Felds in den oberen hinteren Verlängerungsabschnitt und den unteren hinteren Verlängerungsabschnitt durch Halbierung des Verlängerungsabschnitts des hinteren Felds in Artikellängsrichtung unterteilt ist, und

der obere hintere Verlängerungsabschnitt und ein Abschnitt des vorderen Felds in Entsprechung zum oberen hinteren Verlängerungsabschnitt jeweils eine größere Kontraktionsspannung in Artikelseitenrichtung pro Längeneinheit in Artikellängsrichtung als eine Kontraktionsspannung des unteren hinteren Verlängerungsabschnitts haben.

2. Saugfähiger Schlupfartikel nach Anspruch 1, wobei ein unterer Halbabschnitt eines dritten Bereichs jeweils des vorderen Felds und des hinteren Felds eine Kontraktionsspannung in Artikelbreitenrichtung pro Längeneinheit in Artikellängsrichtung hat, die größer als eine Kontraktionsspannung eines ersten Bereichs und kleiner als eine Kontraktionsspannung eines zweiten Bereichs ist, während der Körperabschnitt in drei Bereiche durch Dreiteilung der Seitendichtung in Artikellängsrichtung in den ersten Bereich, den zweiten Bereich und den dritten Bereich nacheinander von einer Seite nahe der Taillenöffnung unterteilt ist.

3. Saugfähiger Schlupfartikel nach Anspruch 1 oder Anspruch 2, wobei der obere hintere Verlängerungsabschnitt und/oder ein Abschnitt des vorderen Felds in Entsprechung zum oberen hinteren Verlängerungsabschnitt eine Kontraktionsspannung in Artikelseitenrichtung pro Längeneinheit in Artikellängsrichtung haben, die kleiner als eine Kontraktionsspannung des zweiten Bereichs des hinteren Felds und/oder des vorderen Felds ist, während der Körperabschnitt in drei Bereiche durch Dreiteilung der Seitendichtung in Längsrichtung in den ersten Bereich, den zweiten Bereich und den dritten Bereich nacheinander von einer Seite nahe der Taillenöffnung unterteilt ist.

4. Saugfähiger Schlupfartikel nach einem der Ansprüche 1 bis 3, wobei der obere hintere Verlängerungsabschnitt und/oder ein Abschnitt des vorderen Felds in Entsprechung zum oberen hinteren Verlängerungsabschnitt eine Kontraktionsspannung in Artikelseitenrichtung pro Längeneinheit in Artikellängsrichtung haben, die gleich einer Kontraktionsspannung eines unteren Halbabschnitts des dritten Bereichs des hinteren Felds und/oder des vorderen Felds ist, während der Körperabschnitt in drei Bereiche durch Dreiteilung der Seitendichtung in Längsrichtung in den ersten Bereich, den zweiten Bereich und den dritten Bereich nacheinander von einer Seite nahe der Taillenöffnung unterteilt ist.

5. Saugfähiger Schlupfartikel nach einem der Ansprüche 1 bis 4, wobei der Verlängerungsabschnitt des hinteren Felds länger als der Verlängerungsabschnitt des vorderen Felds ist.

6. Saugfähiger Schlupfartikel nach einem der Ansprüche 1 bis 5, wobei ein dehnbarer Abschnitt, in dem zwei Bahnen an einer Anzahl getrennt gebildeter Verbindungsabschnitte miteinander verbunden sind und in dem jedes elastische Teil so angeordnet ist, dass es zwischen Verbindungsabschnitten verläuft, die in Artikellängsrichtung benachbart sind, am Körperabschnitt und Verlängerungsabschnitt jeweils des vorderen Felds und des hinteren Felds angeordnet ist.

7. Saugfähiger Schlupfartikel nach einem der Ansprüche 1 bis 6,
wobei ein Verbindungsbereich, in dem die beiden Bahnen mit Kleber verbunden sind, an beiden Seitenkanten jeweils des vorderen Felds und des hinteren Felds oder deren Umgebung angeordnet ist und
der Verbindungsbereich einen Abschnitt mit einer größeren Breite an einem Grenzbereich zwischen dem Körperabschnitt und dem Verlängerungsabschnitt als andere Abschnitte aufweist.

8. Saugfähiger Schlupfartikel nach einem der Ansprüche 1 bis 7,
wobei das vordere Feld und das hintere Feld einen vorderen und einen hinteren Körperabschnitt mit Seitendichtungen an beiden Seitenflächen sowie einen vorderen und einen hinteren Verlängerungsabschnitt aufweisen, die sich vom vorderen bzw. hinteren Körperabschnitt zum Schrittabschnitt ohne Seitendichtungen an beiden Seitenflächen erstrecken,
der hintere Verlängerungsabschnitt eine Länge in Artikellängsrichtung hat, die gleich oder größer als die des vorderen Verlängerungsabschnitts ist,
der vordere Körperabschnitt, der vordere Verlängerungsabschnitt, der hintere Körperabschnitt und ein hinterer entsprechender Abschnitt des hinteren Verlängerungsabschnitts in Entsprechung zum vorderen Verlängerungsabschnitt jeweils Dehnbarkeit in Artikelseitenrichtung aufgrund jedes elastischen Teils haben, das in Artikelseitenrichtung angeordnet ist, und

folgende relationale Ausdrücke (11) bis (14) erfüllt sind unter Definitionen, dass $P_{A1'}$-$P_{A4'}$ Kontraktionsspannungen in Artikelseitenrichtung pro Längeneinheit in Artikellängsrichtung bezeichnen und $Q_{A1'}$-$Q_{A4'}$ Kontraktionsspannungen in Artikelseitenrichtung jeweils pro einzelnes elastisches Teil eines ersten bis dritten Bereichs A1' bis A3' und des vorderen Verlängerungsabschnitts A4' des vorderen Felds bezeichnen, während der vordere Körperabschnitt in drei Bereiche durch Dreiteilung der Seitendichtung in Artikellängsrichtung in den ersten Bereich A1', den zweiten Bereich A2' und den dritten Bereich A3' nacheinander von einer Seite nahe der Taillenöffnung unterteilt ist, sowie Definitionen, dass $P_{B1'}$-$P_{B4'}$ Kontraktionsspannungen in Artikelseitenrichtung pro Längeneinheit in Artikellängsrichtung bezeichnen und $Q_{B1'}$-$Q_{B4'}$ Kontraktionsspannungen in Artikelseitenrichtung jeweils pro einzelnes elastisches Teil eines ersten bis dritten Bereichs B1' bis B3' und des hinteren entsprechenden Abschnitts B4' des hinteren Felds bezeichnen, während der hintere Körperabschnitt in drei Bereiche durch Dreiteilung der Seitendichtung in Artikellängsrichtung in den ersten Bereich B1', den zweiten Bereich B2' und den dritten Bereich B3' nacheinander von einer Seite nahe der Taillenöffnung unterteilt ist:

$$\text{Verhältnis } (P_{A3'}/P_{A1'}) > \text{Verhältnis } (Q_{A3'}/Q_{A1'}), \text{ Verhältnis } (P_{A3'}/P_{A1'}) > 1 \ \dots \ (11)$$

$$\text{Verhältnis } (P_{B3'}/P_{B1'}) > \text{Verhältnis } (Q_{B3'}/Q_{B1'}), \text{ Verhältnis } (P_{B3'}/P_{B1'}) > 1 \ \dots \ (12)$$

$$\text{Verhältnis } (P_{A4'}/P_{A1'}) > \text{Verhältnis } (Q_{A4'}/Q_{A1'}), \text{ Verhältnis } (P_{A4'}/P_{A1'}) > 1 \ \dots \ (13)$$

$$\text{Verhältnis } (P_{B4'}/P_{B1'}) > \text{Verhältnis } (Q_{B4'}/Q_{B1'}), \text{ Verhältnis } (P_{B4'}/P_{B1'}) > 1 \ \dots \ (14)$$

**9.** Saugfähiger Schlupfartikel nach Anspruch 8, wobei folgende relationale Ausdrücke (15) und (16) erfüllt sind:

$$P_{A3'} = P_{A4'}, Q_{A3'} = Q_{A4'} \ \dots \ (15)$$

$$P_{B3'} = P_{B4'}, Q_{B3'} = Q_{B4'} \ \dots \ (16)$$

**10.** Saugfähiger Schlupfartikel nach Anspruch 8 oder Anspruch 9, wobei folgende relationale Ausdrücke (17) und (18) erfüllt sind:

$$4 \text{ cN} < Q_{A3'} < 23 \text{ cN} \ \dots \ (17)$$

$$4 \text{ cN} < Q_{B3'} < 23 \text{ cN} \ \dots \ (18)$$

**11.** Saugfähiger Schlupfartikel nach einem der Ansprüche 8 bis 10, wobei folgende relationale Ausdrücke (19) und (20) erfüllt sind:

$$P_{A2'} > P_{A3'}, P_{B2'} > P_{B3'} \ \dots \ (19)$$

$$P_{A2'} > P_{A4'}, P_{B2'} > P_{B4'} \ \dots \ (20)$$

**12.** Saugfähiger Schlupfartikel nach einem der Ansprüche 8 bis 11, wobei der hintere Verlängerungsabschnitt einen unteren Verlängerungsabschnitt B5' aufweist, der sich vom hinteren entsprechenden Abschnitt zum Schrittabschnitt erstreckt, und der untere Verlängerungsabschnitt Dehnbarkeit in Artikelseitenrichtung aufgrund eines elastischen Teils hat, das in Artikelseitenrichtung angeordnet ist, und ein folgender relationaler Ausdruck (21) unter Definitionen erfüllt ist, dass $P_{B5'}$ eine Kontraktionsspannung des unteren Verlängerungsabschnitts in Artikelseitenrichtung pro Längeneinheit in Artikellängsrichtung bezeichnet und

**EP 2 659 870 B1**

$Q_{B5'}$ eine Kontraktionsspannung in Artikelseitenrichtung jeweils pro einzelnes elastisches Teil des unteren Verlängerungsabschnitts bezeichnet:

$$\text{Verhältnis } (P_{B4'}/P_{B5'}) > \text{Verhältnis } (Q_{B4'}/Q_{B5'}), \text{ Verhältnis } (P_{B4'}/P_{B5'}) > 1 \ldots (21)$$

13. Saugfähiger Schlupfartikel nach einem der Ansprüche 8 bis 12, wobei ein Anordnungsintervall in Artikellängsrichtung der elastischen Teile im zweiten und dritten Bereich A2', A3' und im vorderen Verlängerungsabschnitt A4' des vorderen Felds kleiner als ein Anordnungsintervall in Artikellängsrichtung der elastischen Teile im ersten Bereich A1' des vorderen Felds ist.

14. Saugfähiger Schlupfartikel nach einem der Ansprüche 8 bis 13, wobei ein Anordnungsintervall in Artikellängsrichtung der elastischen Teile im zweiten und dritten Bereich B2', B3' und im hinteren entsprechenden Abschnitt B4' des hinteren Felds kleiner als ein Anordnungsintervall in Artikellängsrichtung der elastischen Teile im ersten Bereich B1' des hinteren Felds ist.

15. Saugfähiger Schlupfartikel nach einem der Ansprüche 8 bis 14,
wobei der hintere Verlängerungsabschnitt einen unteren Verlängerungsabschnitt B5' aufweist, der sich vom hinteren entsprechenden Abschnitt zum Schrittabschnitt erstreckt,
der hintere entsprechende Abschnitt und der untere Verlängerungsabschnitt jeweils Dehnbarkeit in Artikelseitenrichtung aufgrund mehrerer elastischer Teile haben, die in Artikelseitenrichtung angeordnet sind, und
ein Anordnungsintervall in Artikellängsrichtung der elastischen Teile im hinteren entsprechenden Abschnitt größer als ein Anordnungsintervall in Artikellängsrichtung der elastischen Teile im unteren Verlängerungsabschnitt ist.

16. Saugfähiger Schlupfartikel nach einem der Ansprüche 8 bis 15,
wobei der hintere Verlängerungsabschnitt einen unteren Verlängerungsabschnitt B5' aufweist, der sich vom hinteren entsprechenden Abschnitt zum Schrittabschnitt erstreckt,
der erste Bereich des hinteren Körperabschnitts und der untere Verlängerungsabschnitt jeweils Dehnbarkeit in Artikelseitenrichtung aufgrund mehrerer elastischer Teile haben, die in Artikelseitenrichtung angeordnet sind, und
ein Anordnungsintervall in Artikellängsrichtung der elastischen Teile im unteren Verlängerungsabschnitt gleich einem Anordnungsintervall in Artikellängsrichtung der elastischen Teile im ersten Bereich des hinteren Körperabschnitts ist.

17. Saugfähiger Schlupfartikel nach einem der Ansprüche 8 bis 16, wobei ein dehnbarer Abschnitt, in dem zwei Bahnen an einer Anzahl getrennt gebildeter Verbindungsabschnitte miteinander verbunden sind und in dem jedes elastische Teil so angeordnet ist, dass es zwischen Verbindungsabschnitten verläuft, die in Artikellängsrichtung benachbart sind, am Körperabschnitt und Verlängerungsabschnitt jeweils des vorderen Felds und des hinteren Felds angeordnet ist.

**Revendications**

1. Article absorbant à enfiler qui comporte une ouverture pour la taille et deux ouvertures pour les jambes, comprenant :

   un panneau avant rectangulaire qui est adapté de manière à ce qu'il soit porté autour de l'avant du corps de la personne qui le porte ;
   un panneau arrière rectangulaire qui est adapté de manière à ce qu'il soit porté autour de l'arrière du corps de la personne qui le porte ; et
   un assemblage absorbant qui est fixé au panneau avant et au panneau arrière de manière à ce qu'il réalise un pontage de ces deux panneaux ; dans lequel :

   chaque panneau pris parmi le panneau avant et le panneau arrière inclut :

   une partie de corps qui inclut des étanchéités latérales qui sont situées au niveau de deux côtés latéraux des panneaux ; et
   une partie d'extension qui est étendue depuis la partie de corps en direction d'une partie d'entrejambe et qui ne comporte pas d'étanchéités latérales au niveau de deux côtés latéraux des panneaux ;

chaque partie prise parmi la partie de corps et la partie d'extension du panneau avant ainsi que parmi la partie de corps, une partie d'extension arrière supérieure et une partie d'extension arrière inférieure du panneau arrière présente une extensibilité dans la direction latérale de l'article tandis que la partie d'extension du panneau arrière est partitionnée selon des sections que sont la partie d'extension arrière supérieure et la partie d'extension arrière inférieure en bissectant la partie d'extension du panneau arrière dans la direction longitudinale de l'article ; et

chaque partie prise parmi la partie d'extension arrière supérieure et une partie du panneau avant qui correspond à la partie d'extension arrière supérieure présente une contrainte de contraction dans la direction latérale de l'article par longueur unitaire dans la direction longitudinale de l'article qui est plus importante qu'une contrainte de contraction de la partie d'extension arrière inférieure.

2. Article absorbant à enfiler selon la revendication 1, dans lequel une demi-partie inférieure d'une troisième région de chaque panneau pris parmi le panneau avant et le panneau arrière présente une contrainte de contraction dans la direction de largeur de l'article par longueur unitaire dans la direction longitudinale de l'article qui est plus importante qu'une contrainte de contraction d'une première région et qui est plus faible qu'une contrainte de contraction d'une deuxième région, tandis que la partie de corps est partitionnée selon des sections au nombre de trois régions en trissectant l'étanchéité latérale dans la direction longitudinale de l'article de telle sorte que ces trois régions soient la première région, la deuxième région et la troisième région de façon séquentielle depuis un côté proche de l'ouverture pour la taille.

3. Article absorbant à enfiler selon la revendication 1 ou la revendication 2, dans lequel la partie d'extension arrière supérieure et/ou une partie du panneau avant qui correspond à la partie d'extension arrière supérieure présente(nt) une contrainte de contraction dans la direction latérale de l'article par longueur unitaire dans la direction longitudinale de l'article qui est plus faible qu'une contrainte de contraction dans la deuxième région du panneau arrière et/ou du panneau avant, tandis que la partie de corps est partitionnée selon des sections au nombre de trois régions en trissectant l'étanchéité latérale dans la direction longitudinale de telle sorte que ces trois régions soient la première région, la deuxième région et la troisième région de façon séquentielle depuis un côté proche de l'ouverture pour la taille.

4. Article absorbant à enfiler selon l'une quelconque des revendications 1 à 3, dans lequel la partie d'extension arrière supérieure et/ou une partie du panneau avant qui correspond à la partie d'extension arrière supérieure présente(nt) une contrainte de contraction dans la direction latérale de l'article par longueur unitaire dans la direction longitudinale de l'article qui est égale à une contrainte de contraction d'une demi-partie inférieure de la troisième région du panneau arrière et/ou du panneau avant, tandis que la partie de corps est partitionnée selon des sections au nombre de trois régions en trissectant l'étanchéité latérale dans la direction longitudinale de telle sorte que ces trois régions soient la première région, la deuxième région et la troisième région de façon séquentielle depuis un côté proche de l'ouverture pour la taille.

5. Article absorbant à enfiler selon l'une quelconque des revendications 1 à 4, dans lequel la partie d'extension du panneau arrière est plus longue que la partie d'extension du panneau avant.

6. Article absorbant à enfiler selon l'une quelconque des revendications 1 à 5, dans lequel une partie extensible dans laquelle deux feuilles sont jointes de façon mutuelle au niveau d'un certain nombre de parties de jonction qui sont formées de manière discrète et dans laquelle chaque élément élastique est agencé de manière à ce qu'il passe entre des parties de jonction qui sont adjacentes dans la direction longitudinale de l'article est agencée au niveau de la partie de corps et de la partie d'extension de chaque panneau pris parmi le panneau avant et le panneau arrière.

7. Article absorbant à enfiler selon l'une quelconque des revendications 1 à 6, dans lequel :

une région de jonction dans laquelle les deux feuilles sont jointes à l'aide d'un adhésif est agencée au niveau de deux bords latéraux de chaque panneau pris parmi le panneau avant et le panneau arrière ou au niveau de leurs voisinages ; et
la région de jonction inclut une partie qui présente une largeur plus importante au niveau d'une région de frontière entre la partie de corps et la partie d'extension qu'au niveau d'autres parties.

8. Article absorbant à enfiler selon l'une quelconque des revendications 1 à 7, dans lequel :

le panneau avant et le panneau arrière incluent des parties de corps avant et arrière qui incluent des étanchéités latérales au niveau de deux côtés latéraux et des parties d'extension avant et arrière qui s'étendent respectivement depuis les parties de corps avant et arrière en direction de la partie d'entrejambe et qui ne comportent pas d'étanchéités latérales au niveau de deux côtés latéraux ;

la partie d'extension arrière présente une longueur dans la direction longitudinale de l'article qui est la même que celle de la partie d'extension avant ou qui est plus importante que celle-ci ;

chaque partie prise parmi la partie de corps avant, la partie d'extension avant, la partie de corps arrière et une partie correspondante arrière de la partie d'extension arrière qui correspond à la partie d'extension avant présente une extensibilité dans la direction latérale de l'article du fait de chaque élément élastique qui est agencé dans la direction latérale de l'article ; et

les expressions relationnelles (11) à (14) qui suivent sont satisfaites moyennant les définitions consistant en ce que $P_{A1'}$ à $P_{A4'}$ représentent des contraintes de contraction dans la direction latérale de l'article par longueur unitaire dans la direction longitudinale de l'article et $Q_{A1'}$ à $Q_{A4'}$ représentent des contraintes de contraction dans la direction latérale de l'article par élément élastique unique respectivement de première à troisième régions A1' à A3' et de la partie d'extension avant A4' du panneau avant, tandis que la partie de corps avant est partitionnée selon des sections au nombre de trois régions en trissectant l'étanchéité latérale dans la direction longitudinale de l'article de telle sorte que ces trois régions soient la première région A1', la deuxième région A2' et la troisième région A3' de façon séquentielle depuis un côté proche de l'ouverture pour la taille et moyennant les définitions consistant en ce que $P_{B1'}$ à $P_{B4'}$ représentent des contraintes de contraction dans la direction latérale de l'article par longueur unitaire dans la direction longitudinale de l'article et $Q_{B1'}$ à $Q_{B4'}$ représentent des contraintes de contraction dans la direction latérale de l'article par élément élastique unique respectivement de première à troisième régions B1' à B3' et de la partie correspondante arrière B4' du panneau arrière, tandis que la partie de corps arrière est partitionnée selon des sections au nombre de trois régions en trissectant l'étanchéité latérale dans la direction longitudinale de l'article de telle sorte que ces trois régions soient la première région B1', la deuxième région B2' et la troisième région B3' de façon séquentielle depuis un côté proche de l'ouverture pour la taille :

$$\text{Rapport } (P_{A3'}/P_{A1'}) > \text{Rapport } (Q_{A3'}/Q_{A1'}), \quad \text{Rapport } (P_{A3'}/P_{A1'}) > 1 \qquad (11)$$

$$\text{Rapport } (P_{B3'}/P_{B1'}) > \text{Rapport } (Q_{B3'}/Q_{B1'}), \quad \text{Rapport } (P_{B3'}/P_{B1'}) > 1 \qquad (12)$$

$$\text{Rapport } (P_{A4'}/P_{A1'}) > \text{Rapport } (Q_{A4'}/Q_{A1'}), \quad \text{Rapport } (P_{A4'}/P_{A1'}) > 1 \qquad (13)$$

$$\text{Rapport } (P_{B4'}/P_{B1'}) > \text{Rapport } (Q_{B4'}/Q_{B1'}), \quad \text{Rapport } (P_{B4'}/P_{B1'}) > 1 \qquad (14).$$

9. Article absorbant à enfiler selon la revendication 8, dans lequel les expressions relationnelles (15) et (16) qui suivent sont satisfaites :

$$P_{A3'} = P_{A4'}, Q_{A3'} = Q_{A4'} \qquad (15)$$

$$P_{B3'} = P_{B4'}, Q_{B3'} = Q_{B4'} \qquad (16).$$

10. Article absorbant à enfiler selon la revendication 8 ou la revendication 9, dans lequel les expressions relationnelles (17) et (18) qui suivent sont satisfaites :

$$4 \text{ cN} < Q_{A3'} < 23 \text{ cN} \qquad (17)$$

$$4 \text{ cN} < Q_{B3'} < 23 \text{ cN} \qquad (18).$$

11. Article absorbant à enfiler selon l'une quelconque des revendications 8 à 10, dans lequel les expressions relationnelles (19) et (20) qui suivent sont satisfaites :

$$P_{A2'} > P_{A3'}, \; P_{B2'} > P_{B3'} \qquad\qquad (19)$$

$$P_{A2'} > P_{A4'}, \; P_{B2'} > P_{B4'} \qquad\qquad (20).$$

**12.** Article absorbant à enfiler selon l'une quelconque des revendications 8 à 11, dans lequel :

la partie d'extension arrière inclut une partie d'extension inférieure B5' qui est étendue depuis la partie correspondante arrière en direction de la partie d'entrejambe, la partie d'extension inférieure présente une extensibilité dans la direction latérale de l'article du fait d'un élément élastique qui est agencé dans la direction latérale de l'article ; et
une expression relationnelle (21) qui suit est satisfaite moyennant les définitions consistant en ce que $P_{B5'}$ représente une contrainte de contraction de la partie d'extension inférieure dans la direction latérale de l'article par longueur unitaire dans la direction longitudinale de l'article et $Q_{B5'}$ représente une contrainte de contraction dans la direction latérale de l'article par élément élastique unitaire, de façon respective, de la partie d'extension inférieure :

$$\text{Rapport } (P_{B4'}/P_{B5'}) > \text{Rapport } (Q_{B4'}/Q_{B5'}), \quad \text{Rapport } (P_{B4'}/P_{B5'}) > 1 \qquad (21).$$

**13.** Article absorbant à enfiler selon l'une quelconque des revendications 8 à 12, dans lequel un intervalle d'agencement dans la direction longitudinale de l'article des éléments élastiques dans les deuxième et troisième régions A2', A3' et dans la partie d'extension avant A4' du panneau avant est plus petit qu'un intervalle d'agencement dans la direction longitudinale de l'article des éléments élastiques dans la première région A1' du panneau avant.

**14.** Article absorbant à enfiler selon l'une quelconque des revendications 8 à 13, dans lequel un intervalle d'agencement dans la direction longitudinale de l'article des éléments élastiques dans les deuxième et troisième régions B2', B3' et dans la partie correspondante arrière B4' du panneau arrière est plus petit qu'un intervalle d'agencement dans la direction longitudinale de l'article des éléments élastiques dans la première région B1' du panneau arrière.

**15.** Article absorbant à enfiler selon l'une quelconque des revendications 8 à 14, dans lequel :

la partie d'extension arrière inclut une partie d'extension inférieure B5' qui est étendue depuis la partie correspondante arrière en direction de la partie d'entrejambe ;
chaque partie prise parmi la partie correspondante arrière et la partie d'extension inférieure présente une extensibilité dans la direction latérale de l'article du fait d'une pluralité d'éléments élastiques qui sont agencés dans la direction latérale de l'article ; et
un intervalle d'agencement dans la direction longitudinale de l'article des éléments élastiques dans la partie correspondante arrière est plus grand qu'un intervalle d'agencement dans la direction longitudinale de l'article des éléments élastiques dans la partie d'extension inférieure.

**16.** Article absorbant à enfiler selon l'une quelconque des revendications 8 à 15, dans lequel :

la partie d'extension arrière inclut une partie d'extension inférieure B5' qui est étendue depuis la partie correspondante arrière en direction de la partie d'entrejambe ;
chaque section prise parmi la première région de la partie de corps arrière et la partie d'extension inférieure présente une extensibilité dans la direction latérale de l'article du fait d'une pluralité d'éléments élastiques qui sont agencés dans la direction latérale de l'article ; et
un intervalle d'agencement dans la direction longitudinale de l'article des éléments élastiques dans la partie d'extension inférieure est égal à un intervalle d'agencement dans la direction longitudinale de l'article des éléments élastiques dans la première région de la partie de corps arrière.

**17.** Article absorbant à enfiler selon l'une quelconque des revendications 8 à 16, dans lequel une partie extensible dans laquelle deux feuilles sont jointes de façon mutuelle au niveau d'un certain nombre de parties de jonction qui sont formées de manière discrète et dans laquelle chaque élément élastique est agencé de manière à ce qu'il passe entre des parties de jonction qui sont adjacentes dans la direction longitudinale de l'article est agencée au niveau de la partie de corps et de la partie d'extension de chaque panneau pris parmi le panneau avant et le panneau arrière.

FIG. 1

EP 2 659 870 B1

FIG. 2

42

FIG. 3

EP 2 659 870 B1

FIG. 4

FIG. 5(a)

FIG. 5(b)

FIG. 6

FIG. 7

EP 2 659 870 B1

FIG. 8

EP 2 659 870 B1

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008508082 A
- JP 2008178682 A
- JP 2008212249 A
- JP 2006061680 A